# EUROPEAN PATENT SPECIFICATION

(11) **EP 3 130 553 B1**
(45) Date of publication and mention of the grant of the patent: **17.10.2018**
(21) Application number: 16183214.2
(22) Date of filing: 08.08.2016
(51) Int. Cl.: B65H 63/06, D01H 13/22, G01N 33/36, G01N 21/89

(54) **YARN MONITORING DEVICE AND YARN WINDING MACHINE**
GARNÜBERWACHUNGSVORRICHTUNG UND GARNWICKELMASCHINE
DISPOSITIF DE SURVEILLANCE DE FIL ET MACHINE DE BOBINAGE DE FIL

(30) Priority: 12.08.2015 JP 2015159497
(43) Date of publication of application: 15.02.2017
(73) Proprietor: Murata Machinery, Ltd., Minami-ku Kyoto-shi Kyoto 601-8326 (JP)
(72) Inventor: OKAZAKI, Yohei, Kyoto, 612-8686 (JP); MATSUMOTO, Keigo, Kyoto, 612-8686 (JP)
(74) Representative: Schenk, Markus

(56) References cited:
- DE-A1-102014 220 761
- JP-A- H11 322 196
- US-A1- 2003 070 481

## Description

### BACKGROUND OF THE INVENTION

### 1. Field of the Invention

The present invention relates to a yarn monitoring device and a yarn winding machine.

### 2. Description of the Related Art

A yarn monitoring device such as a yarn clearer is provided in yarn winding machines to detect a state of a yarn such as a thickness of the yarn. As such a yarn monitoring device, a device including a holder on which is mounted a sensor part for measurement for measuring the state of the yarn is known in the art (e.g., see Japanese Patent Application Laid-Open No. H11-322196). In the device described in Japanese Patent Application Laid-Open No. H11-322196, each of a plurality of holders (measurement cells) is fixed to and supported by each of a plurality of flat substrates (printed substrates). A processor is mounted on each of the substrates, and a signal outputted from the sensor part for measurement mounted on each of the holders is processed respectively by the processor.

DE 10 2014 220761 A1 discloses a yarn winding machine comprising a holder. The holder has a first portion and a second portion and a plurality of detection portions arranged along a yarn travel path. A circuit board is coupled to the detection portions via flexible wiring elements.

### SUMMARY OF THE INVENTION

In the above-explained yarn monitoring device, because the flat substrate and the holder are fixed to each other by soldering and the like, the holder and the flat substrate can only be replaced together as one unit. That is, it is not easy to replace or to remove the holder and the flat substrate separately. Accordingly, changing to an appropriate holder to be used, and appropriately changing the arrangement of the flat substrate depending on the used holder are difficult. Therefore, there is a possibility that it may be difficult to cope with a specification change of the holder.

The present invention has been made in view of the above discussion and it is an object thereof to provide a yarn monitoring device and a yarn winding machine that can easily cope with the specification change of the holder.

A yarn monitoring device according to an aspect of the present invention includes a plurality of holders that are piled up one above the other along a predetermined direction and through which a traveling yarn passes; at least one substrate; and a case that accommodates the holders and the at least one substrate, wherein a sensor part for measurement that is configured to measure a state of the traveling yarn is mounted on at least one of the holders, the at least one substrate is associated with the holder on which the sensor part for measurement is mounted, and the at least one substrate is configured to process a signal received from the sensor part for measurement mounted on the holder, and the sensor part for measurement is electrically connected to the at least one substrate via a flexible connecting part. Two adjacent holders among the holders are detachably coupled to each other with engagement of concave and convex parts.

A yarn monitoring device according to another aspect of the present invention includes a plurality of holders that are piled up one above the other along a predetermined direction and through which a traveling yarn passes; a plurality of substrates; and a case that accommodates the holders and the substrates. A sensor part for measurement that measures a state of the traveling yarn is mounted on each of the holders. One or more of the substrates are arranged corresponding to each of the holders and the substrates process a signal received from the sensor part for measurement mounted on a corresponding holder. The sensor part for measurement in each of the holders is electrically connected to the corresponding one or more substrates via a flexible connecting part.

In the above yarn monitoring device, one or more substrates are provided corresponding to the sensor part for measurement mounted on each of the holders, and these substrates are electrically connected to the sensor part for measurement via flexible connecting parts. Accordingly, only the holder or only the substrate can be easily replaced or removed. Moreover, the holder to be used can be appropriately changed, and, the arrangement of the substrate to be used can be appropriately changed depending on the used holder. As a result, it is possible to easily cope with the specification change of the holder.

A yarn monitoring device according to another aspect of the present invention includes a plurality of holders that are piled up one above the other along a predetermined direction and through which a traveling yarn passes; one or more substrates; and a case that accommodates the holders and the substrates. A part of the holders is a sensor mounting holder on which a sensor part for measurement that measures a state of the traveling yarn is mounted. Remaining part of the holders is a sensor not-mounted holder on which no sensor part for measurement is mounted. One or more of the substrates are arranged corresponding to each of the sensor mounting holders, and the substrates process a signal received from the sensor part for measurement mounted on the corresponding sensor mounting holder.

In the above yarn monitoring device, a part of the holders constitutes the sensor mounting holder and other part of the holders constitutes the sensor not-mounted holder (that is, a dummy holder). As a result, the case having the same configuration as for a specification that requires all of the holders (that is, when all the holders are the sensor mounting holders) can be used even in a specification that requires only a part of the holders. As a result, it is possible to easily cope with the specification change of the holder.

In the above yarn monitoring device, two adjacent holders among the holders can be detachably coupled to each other. According to this configuration, a part of the holders can be easily changed.

In embodiments of the above yarn monitoring device, an end holder among the holders arranged at an end in the predetermined direction includes a first positioning member that positions the end holder with respect to the case, the case includes a second positioning member that directly or indirectly positions the end holder with respect to the case, and in a coupled state, the holders are held by a first casing section and a second casing section in a direction parallel to the predetermined direction and the holders are positioned in the case by the first positioning member and the second positioning member. In embodiments corresponding end holders are arranged at either ends in the predetermined direction. According to the above configuration, the holders can be held together in the direction parallel to the predetermined direction by coupling the second casing section to the first casing section, and the holders can be positioned by using the first positioning member and the second positioning member. Therefore, the holders can be arranged with high positioning accuracy and good workability.

Embodiments of the above yarn monitoring device can further include a plurality of yarn path guides that guides the traveling yarn. At least one end yarn path guide arranged at an end in the predetermined direction among the yarn path guides is arranged between the end holders and the case. In embodiments, corresponding end yarn path guides are arranged at either ends in the predetermined direction. The yarn path guides include a third positioning member that positions the yarn path guides with respect to the case and the holders. The holders, the case, and the yarn path guides are positioned by the first positioning member, the second positioning member, and the third positioning member. According to this configuration, the yarn path guides can be arranged with high positioning accuracy.

In embodiments of the yarn monitoring device, one of the first positioning member and the second positioning member can be a convex part, the other of the first positioning member and the second positioning member can be a notch in which the convex part engages, and the third positioning member can be a through-hole for inserting the convex part, or a cutout portion in which the convex part engages. According to this configuration, the positioning of the holders, the case, and the yarn path guides can be implemented with a simple configuration and efficiently.

In the yarn monitoring device, two adjacent holders among the holders are detachably coupled to each other with engagement of concave and convex parts. According to this configuration, when coupling the holders to each other, the need to perform an adhesion work can be eliminated.

Embodiments of the above yarn monitoring device can further include a rigid flexible substrate, which is different from the substrates, including a plurality of rigid parts and a plurality of flexible parts. The sensor part for measurement can be mounted on each of the rigid parts. The flexible parts can electrically connect at least two of the rigid parts to each other. The rigid parts and the flexible parts can be arranged surrounding a traveling area of the yarn when seen from the predetermined direction. According to the above configuration, the rigid flexible substrate can be arranged in a compact manner.

In embodiments of the above yarn monitoring device, the holders can include a first holder that constitutes a first detection module having at least a function to detect a thickness of the yarn, and a second holder that constitutes a second detection module having at least a function to detect a foreign substance contained in the yarn. According to the above configuration, a foreign substance contained in the yarn and a thickness of the yarn can be monitored.

In embodiments of the above yarn monitoring device, the substrate can be a flat substrate. Moreover, in the above yarn monitoring device, the case can include at least the first casing section and the second casing section couplable to the first casing section, and the case can accommodate the holders in the coupled state in which the second casing section is coupled to the first casing section.

A yarn winding machine according to still another aspect of the present invention that winds a yarn to form a package, includes the above yarn monitoring device, and the predetermined direction is a traveling direction of the yarn.

According to the above yarn winding machine, it is possible to easily cope with the specification change of the holder in the yarn monitoring device.

According to the present invention, it is possible to provide a yarn monitoring device and a yarn winding machine that can easily cope with the specification change of the holder.

### BRIEF DESCRIPTION OF THE DRAWINGS

FIG. 1 is a front view of a spinning machine according to a first embodiment.
FIG. 2 is a side view of a spinning unit included in the spinning machine shown in FIG. 1.
FIG. 3 is a perspective view of a yarn monitoring device according to the first embodiment.
FIG. 4 is an exploded perspective view of the yarn monitoring device shown in FIG. 3.
FIG. 5 is a perspective view of an internal structure of the yarn monitoring device shown in FIG. 3.
FIG. 6 is another perspective view of an internal structure of the yarn monitoring device shown in FIG. 3.
FIG. 7 is a perspective view of a first holder included in the yarn monitoring device shown in FIG. 3.
FIG. 8 is another perspective view of the first holder shown in FIG. 7.
FIG. 9 is a plan view of the first holder shown in FIG. 7.
FIG. 10 is a bottom view of the first holder shown in FIG. 7.
FIG. 11 is a plan view indicating a sensor part for measurement mounted on the first holder shown in FIG. 7.
FIG. 12 is a perspective view of a second holder included in the yarn monitoring device shown in FIG. 3.
FIG. 13 is another perspective view of the second holder shown in FIG. 12.
FIG. 14 is a plan view of the second holder shown in FIG. 12.
FIG. 15 is a bottom view of the second holder shown in FIG. 12.
FIG. 16 is a plan view indicating a sensor part for measurement mounted on the second holder shown in FIG. 12.
FIG. 17A is a perspective view of a yarn path guide included in the yarn monitoring device shown in FIG. 3, and FIG. 17B is another perspective view of the yarn path guide in the yarn monitoring device shown in FIG. 3.
FIG. 18 is a perspective view of an upper casing of the yarn monitoring device shown in FIG. 3.
FIG. 19 is a perspective view of a lower casing of the yarn monitoring device shown in FIG. 3.
FIG. 20 is a longitudinal cross-sectional view of the yarn monitoring device shown in FIG. 3.
FIG. 21 is a perspective view of an internal structure of a yarn monitoring device according to a second embodiment.

### DETAILED DESCRIPTION

Exemplary embodiments of the present invention are explained below in detail with reference to the accompanying drawings. Identical components or corresponding components are indicated by the same reference symbols in the drawings and redundant explanation thereof is omitted.

### First Embodiment

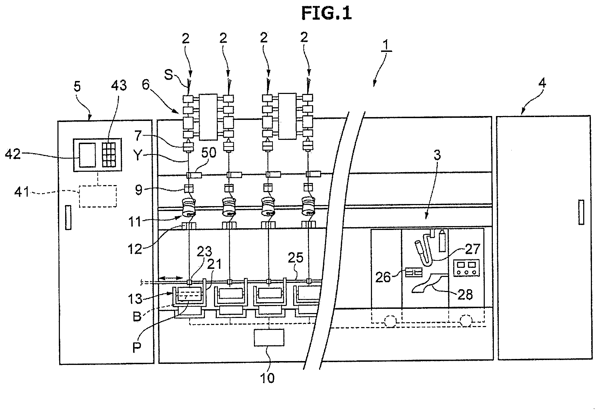

To begin with, a configuration of a spinning machine (a yarn winding machine) 1 including a yarn monitoring device 50 according to the present embodiment is explained below with reference to FIG. 1. In the below explanation, "upstream" and "downstream" refer to upstream and downstream in a traveling direction of a yarn Y during spinning.

As shown in FIG. 1, the spinning machine 1 includes a plurality of spinning units 2, a yarn jointing cart 3, a not-shown doffing cart, a first-end frame 4, and a second-end frame 5. The spinning units 2 are arranged side-by-side. Each of the spinning units 2 generates the yarn Y and winds the yarn Y into a package P. The yarn jointing cart 3 performs a yarn jointing operation in a certain spinning unit 2 in which the yarn Y is cut or becomes discontinuous for some reason. When the package P becomes fully wound in a certain spinning unit 2, the doffing cart doffs the fully wound package P and supplies a new empty bobbin B to that spinning unit 2.

Devices such as a collecting device for collecting fiber waste, yarn waste, and the like produced in the spinning unit 2 are accommodated in the first-end frame 4. An air supplying section that supplies air to various elements of the spinning machine 1 after appropriately adjusting an air pressure of compressed air (air) supplied to the spinning machine 1, and a driving motor and the like that supplies driving power to various elements of the spinning unit 2 are arranged in the second-end frame 5. The second-end frame 5 is provided with a machine-frame controlling device 41, a display screen 42, and one or more input keys 43. The machine-frame controlling device 41 centrally manages and controls various elements of the spinning machine 1. The display screen 42 displays information and the like about setting contents and / or a state of the spinning unit 2. An operator can perform various settings of the spinning unit 2 by appropriately operating the input keys 43.

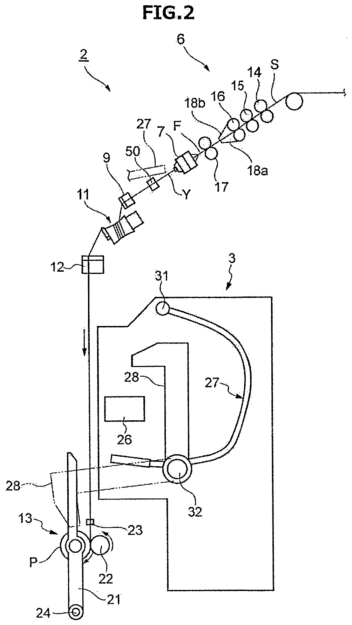

As shown in FIGS. 1 and 2, each of the spinning units 2 includes a drafting device 6, an air spinning device 7, the yarn monitoring device 50, a tension sensor 9, a yarn accumulating device 11, a waxing device 12, and a winding device 13 in this order from upstream in a traveling direction of the yarn Y. One unit controller 10 is arranged for a predetermined number of the spinning units 2, and the unit controller 10 controls operations of those spinning units 2.

The drafting device 6 drafts a sliver (a fiber bundle) S. The drafting device 6 includes a back roller pair 14, a third roller pair 15, a middle roller pair 16, and a front roller pair 17 in this order from upstream in a traveling direction of the sliver S. Each of the roller pairs 14, 15, 16, and 17 includes a bottom roller and a top roller. The bottom roller is rotationally driven by the driving motor arranged in the second-end frame 5 or a driving motor arranged in each of the spinning units 2. An apron belt 18a is stretched over the bottom roller of the middle roller pair 16. An apron belt 18b is stretched over the top roller of the middle roller pair 16.

The air spinning device 7 generates the yarn Y by applying twists by using a swirling air current to a fiber bundle F drafted by the drafting device 6. More particularly, although not shown, the air spinning device 7 includes a spinning chamber, a fiber guiding section, swirling air current generating nozzles, and a hollow guiding shaft. The fiber guiding section guides the fiber bundle F supplied from the drafting device 6, which is arranged upstream, to the inside of the spinning chamber. The swirling air current generating nozzles are arranged surrounding a path on which the fiber bundle F travels and produce a swirling air current inside the spinning chamber. The fiber ends of the fibers constituting the fiber bundle F are reversed and whirled by this swirling air current. The hollow guiding shaft guides the yarn Y from the inside of the spinning chamber to the outside of the air spinning device 7.

The yarn monitoring device 50 monitors, at a location between the air spinning device 7 and the yarn accumulating device 11, information about the traveling yarn Y, and detects presence / absence of a yarn defect in the yarn Y based on the monitored information. Upon detecting the yarn defect, the yarn monitoring device 50 transmits a yarn-defect detection signal to the unit controller 10. The yarn monitoring device 50 detects, for example, a thickness abnormality of the yarn Y and / or a foreign substance contained in the yarn Y as the yarn defect. The yarn monitoring device 50 also detects a yarn breakage and the like. The tension sensor 9 measures, at a location between the air spinning device 7 and the yarn accumulating device 11, a tension of the traveling yarn Y, and transmits a tension measurement signal representing the measured tension to the unit controller 10. When the unit controller 10 determines, based on a detection result obtained in the yarn monitoring device 50 and / or the tension sensor 9, that an abnormality is present in the yarn Y, the yarn Y is cut in that spinning unit 2. Specifically, the yarn Y is cut by suspending the generation of the yarn Y by stopping the supply of the air to the air spinning device 7. Alternatively, the yarn Y can be cut by using a dedicated cutter.

The waxing device 12 applies wax to the yarn Y at a location between the yarn accumulating device 11 and the winding device 13.

The yarn accumulating device 11 removes a slack of the yarn Y at a location between the air spinning device 7 and the winding device 13. The yarn accumulating device 11 has the following functions: to stably pull the yarn Y from the air spinning device 7, to prevent slacking of the yarn Y by temporarily accumulating the yarn Y fed out from the air spinning device 7 when the yarn jointing cart 3 performs the yarn jointing operation and the like, and to prevent variations in the tension of the yarn Y downstream of the yarn accumulating device 11 from being conveyed to the air spinning device 7.

The winding device 13 winds the yarn Y around the bobbin B thereby forming the package P. The winding device 13 includes a cradle arm 21, a winding drum 22, and a traversing guide 23. The cradle arm 21 rotatably supports the bobbin B. The cradle arm 21 is pivotably supported by a support shaft 24. By pivoting the cradle arm 21, a surface of the bobbin B or a surface of the package P can be caused to come into contact with a surface of the winding drum 22 at an appropriate pressure. The not-shown driving motor provided in the second-end frame 5 drives the winding drums 22 of a predetermined number of the spinning units 2 all at once. When the winding drums 22 of the corresponding spinning units 2 are rotated, the bobbin B or the package P is rotated in a direction that allows winding the yarn Y into the package P. The traversing guides 23 of a predetermined number of the spinning units 2 are arranged on a shaft 25 that is shared by those spinning units 2. The traversing guide 23 traverses the yarn Y by a predetermined width on the rotating bobbin B or the package P when the driving motor arranged in the second-end frame 5 causes the shaft 25 to perform a reciprocating movement along a direction of a rotation axis of the winding drum 22.

The yarn jointing cart 3 travels to and performs a yarn jointing operation in a certain spinning unit 2 in which the yarn Y is cut or becomes discontinuous for some reason. The yarn jointing cart 3 includes a yarn jointing device 26, a suction pipe 27, and a suction mouth 28. The suction pipe 27 is pivotably supported by a support shaft 31. The suction pipe 27 catches the yarn Y from the air spinning device 7 and guides the caught yarn Y to the yarn jointing device 26. The suction mouth 28 is pivotably supported by a support shaft 32. The suction mouth 28 catches the yarn Y from the winding device 13 and guides the caught yarn Y to the yarn jointing device 26. The yarn jointing device 26 joints the yarns Y guided thereto. The yarn jointing device 26 can be a splicer that uses compressed air, a piecer that uses a seed yarn, a knotter that mechanically joints the yarn Y, and the like. In the following explanation, the side on which a yarn end of the yarn Y from the winding device 13 is guided by the suction mouth 28 shall be called a "front side", and the side on the opposite side of this side shall be called a "back side".

When the yarn jointing cart 3 performs the yarn jointing operation, the package P is rotated in a direction that allows unwinding of the yarn Y from the package P (reverse rotation). When doing so, the cradle arm 21 is moved by a not-shown air cylinder so that the package P separates from the winding drum 22, and the package P is caused to perform the reverse rotation by a not-shown reverse-rotation roller arranged in the yarn jointing cart 3.

The yarn monitoring device 50 according to the first embodiment is explained below in greater detail.

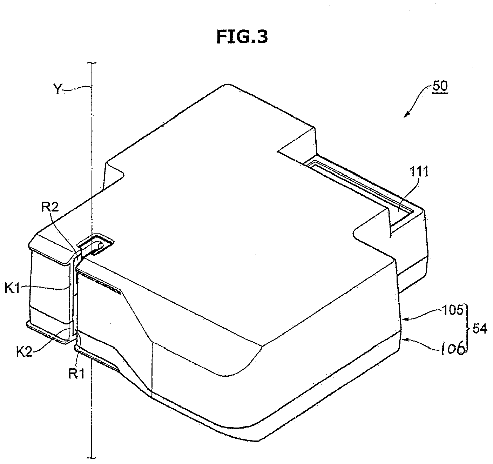

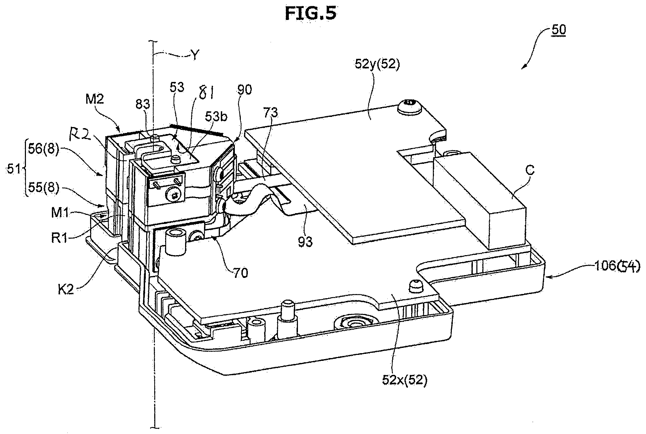

As shown in FIGS. 3 to 6, the yarn monitoring device 50 includes a holder coupler 51, a plurality of flat substrates 52, yarn path guides 53, and a case 54. The holder coupler 51 includes a first holder 55 and a second holder 56 as a plurality of holders 8 that are piled up one above the other along the traveling direction (a predetermined direction) of the yarn Y. A configuration of these components is explained below.

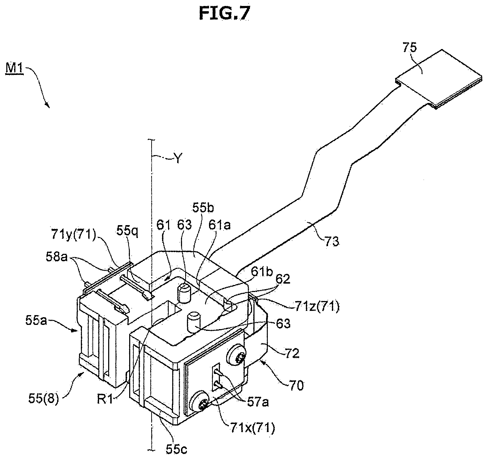

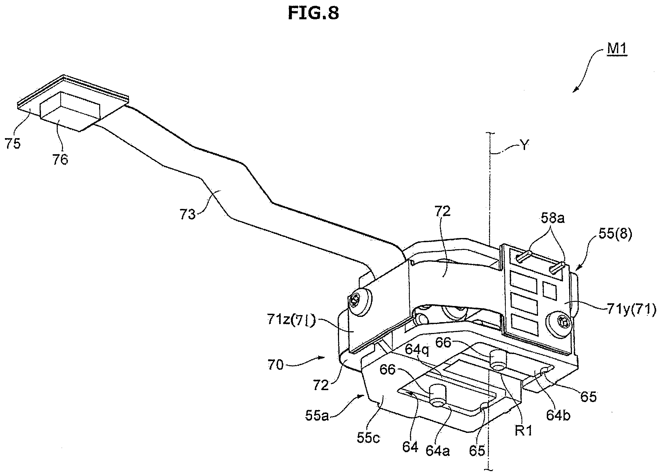

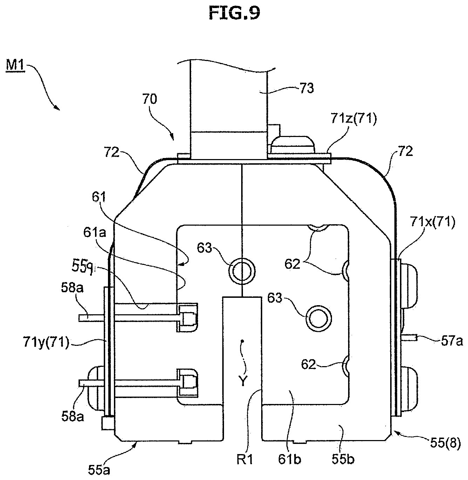

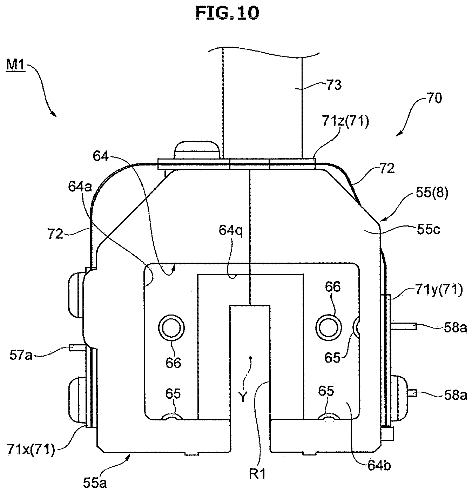

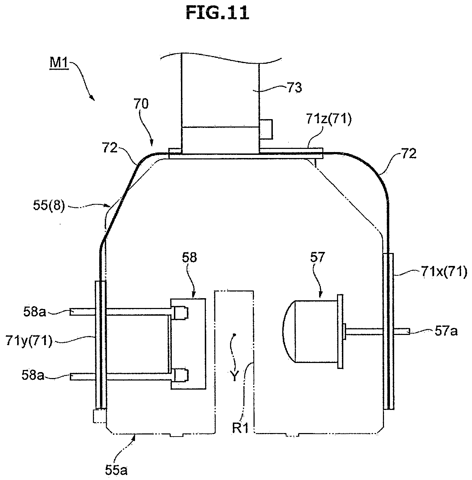

As shown in FIGS. 7 to 11, the first holder 55 constitutes a first detection module M1 through which the traveling yarn Y is passed to detect the state of the yarn Y. The first detection module M1 has a yarn thickness detection function to detect a thickness of the traveling yarn Y. The first holder 55 is arranged downstream of the second holder 56. The order in which the first holder 55 and the second holder 56 are arranged is not limited, and the first holder 55 can be arranged upstream of the second holder 56. Among the holders 8, the first holder 55 is arranged at one end in the traveling direction of the yarn Y. The first holder 55 includes a body 55a that is made of, for example, resin. The first holder 55 is separable into a left part and a right part (in a left-right direction that is the travelling direction of the yarn Y and a direction that is orthogonal to the front-back direction). Thus, the first holder 55 is constituted by a pair of separable parts that is a combination of the left part and the right part. However, the first holder 55 can be an integral body that cannot be separated into the left part and the right part.

The body 55a of the first holder 55 is provided with a traveling area R1 through which the yarn Y travels. The traveling area R1 is a space that extends along a traveling path of the yarn Y. The traveling area R1 is open on the front side. In different words, the traveling area R1 is a passage through which the traveling yarn Y passes, and it is formed such that it extends from upstream to downstream and has a groove shape that is open on the front side. When seen from the traveling direction of the yarn Y, the traveling area R1 is formed in a U-shape.

An upstream concave part 61 is formed in a top surface 55b that is an upstream end surface of the body 55a of the first holder 55. The upstream concave part 61 is formed in a rectangular shape in an inner area from an outer margin of the top surface 55b. When seen from the traveling direction of the yarn Y, the upstream concave part 61 is formed on all sides except on the side on which the traveling area R1 is open.

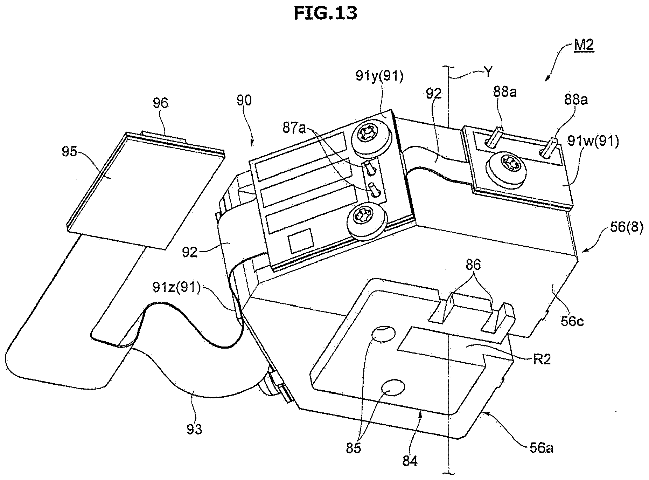

The upstream concave part 61 engages with a later-explained downstream convex part 84 (see FIG. 13) of the second holder 56. When seen from the traveling direction of the yarn Y, the upstream concave part 61 has a cross-sectional shape that corresponds with the downstream convex part 84. The upstream concave part 61 has a depth that is equal to or more than a projecting height of the downstream convex part 84. A plurality of ribs 62 is arranged on an inner side surface 61a of the upstream concave part 61. The ribs 62 project inward from the side surface 61a so as to bulge. When the downstream convex part 84 is engaged with the upstream concave part 61, the ribs 62 remove a gap (so-called "wobble", also called as "backlash" or "play") between the upstream concave part 61 and the downstream convex part 84.

A plurality of (in the present embodiment, two) upstream convex parts 63 is arranged on a bottom surface 61b inside the upstream concave part 61. The upstream convex parts 63 protrude further upstream of the top surface 55b. The upstream convex parts 63 engage with later-explained holes 85 (see FIG. 13) formed in the second holder 56. When seen from the traveling direction of the yarn Y, the upstream convex parts 63 have a cross-sectional shape that corresponds with the holes 85. The upstream convex parts 63 shown in the drawings have a circular cross-sectional shape.

A downstream concave part 64 is formed in a bottom surface 55c that is a downstream end surface of the body 55a of the first holder 55. The downstream concave part 64 is formed in a rectangular shape in an inner area from an outer margin of the bottom surface 55c. When seen from the traveling direction of the yarn Y, the downstream concave part 64 is formed on all sides except on the side on which the traveling area R1 is open.

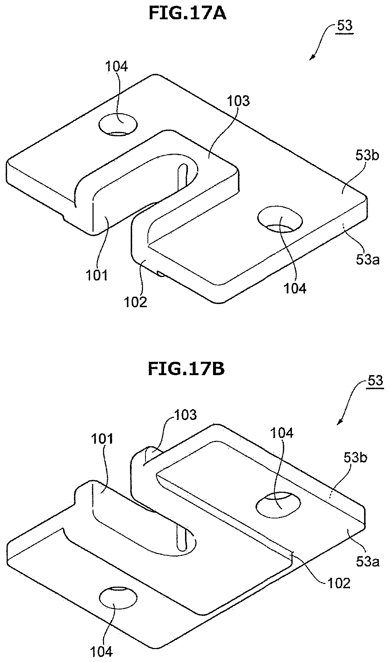

The downstream concave part 64 is a space in which the yarn path guide 53 is arranged. When seen from the traveling direction of the yarn Y, the downstream concave part 64 has a shape that corresponds with the yarn path guide 53. The downstream concave part 64 has a depth that corresponds with a thickness of the yarn path guide 53. A plurality of ribs 65 is arranged on an inner side surface 64a of the downstream concave part 64. The ribs 65 project inward from the side surface 64a so as to bulge. When the yarn path guide 53 is engaged with the downstream concave part 64, the ribs 65 remove a gap (so-called "wobble", also called as "backlash" or "play") between the yarn path guide 53 and the downstream concave part 64. A step 64q that deepens in a rectangle shape is formed around the traveling area R1 in a bottom surface 64b of the downstream concave part 64. The step 64q engages with a later-explained convex part 102 (see FIG. 17) of the yarn path guide 53.

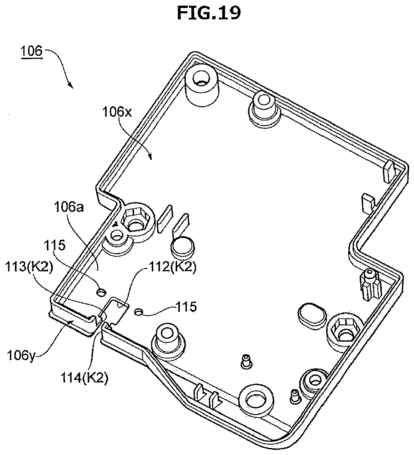

A plurality of downstream convex parts 66 is arranged on the bottom surface 64b of the downstream concave part 64. One downstream convex part 66 is arranged on one side and another downstream convex part 66 is arranged on the other side of the traveling area R1 on the bottom surface 64b. The downstream convex parts 66 protrude further downstream of the bottom surface 55c. The downstream convex parts 66 engage with later-explained notches 115 (see FIG. 19) of the case 54. When seen from the traveling direction of the yarn Y, the downstream convex parts 66 have a cross-sectional shape that corresponds with the notches 115. The downstream convex parts 66 shown in the drawings have a circular cross-sectional shape. The downstream convex parts 66 constitute a first positioning member for positioning the first holder 55 with respect to the case 54.

The first holder 55 is a sensor mounting holder for mounting a sensor part for measurement. A light emitting element 57 and a light receiving element 58 that detect the state of the yarn Y traveling in the traveling area R1 are mounted inside the body 55a of the first holder 55. The body 55a supports the light emitting element 57 and the light receiving element 58. The light emitting element 57 and the light receiving element 58 constitute the sensor part for measurement. The light emitting element 57 and the light receiving element 58 are arranged across the traveling area R1 facing each other. A light emitting diode (LED) and the like, for example, can be used as the light emitting element 57. A photodiode and the like, for example, can be used as the light receiving element 58.

The light emitting element 57 has a pair of lead pins 57a. Tips of the lead pins 57a extend away from the traveling area R1. The light receiving element 58 has a pair of lead pins 58a. Tips of the lead pins 58a extend away from the traveling area R1 through a groove 55q formed in the top surface 55b. In the first detection module M1 including the first holder 55 on which the light emitting element 57 and the light receiving element 58 are mounted, a light is emitted from the light emitting element 57 on the yarn Y traveling in the traveling area R1, and a transmitted light that passes through the yarn Y among the emitted light is received by the light receiving element 58. The thickness of the yarn Y is determined based on an amount of the transmitted light received by the light receiving element 58.

The yarn monitoring device 50 includes a rigid flexible substrate 70. The rigid flexible substrate 70 includes a plurality of rigid parts 71, a plurality of flexible parts 72, and a flexible connecting part 73. The rigid flexible substrate 70 constitutes the first detection module M1.

Rigid material such as glass epoxy is used for the rigid parts 71. A material having high flexibility and bendability such as polyimide is used for the flexible parts 72 and the flexible connecting part 73. If the same material is used for the rigid parts 71, the flexible parts 72, and the flexible connecting part 73, the rigid parts 71 can be made thicker than the flexible parts 72 and the flexible connecting part 73. In short, it is sufficient that the flexibility and bendability of the flexible parts 72 and the flexible connecting part 73 are higher than the flexibility and bendability of the rigid parts 71. It is sufficient that the rigidity of the rigid parts 71 is higher than that of the flexible parts 72 and the flexible connecting part 73.

The rigid part 71 is a rigid substrate provided with a wiring pattern, through holes, and the like. The rigid parts 71 are fixed to side surfaces of the first holder 55. Specifically, in the body 55a of the first holder 55, one rigid part 71 is fixed with one or more screws to a back side surface and to each of a pair of side surfaces that is located across the traveling area R1. Alternatively, the rigid parts 71 can be fixed to the body 55a by welding. The lead pins 57a of the light emitting element 57 are connected by soldering and the like to a rigid part 71x on the light emitting element 57 side among the rigid parts 71. The lead pins 58a of the light receiving element 58 are connected by soldering and the like to a rigid part 71y on the light receiving element 58 side among the rigid parts 71. That is, the light emitting element 57 and the light receiving element 58 are mounted on the rigid parts 71x and 71y, respectively. The rigid parts 71 have a plate shaped form that extends along the traveling direction of the yarn Y.

The flexible part 72 is a flexible substrate provided with a wiring pattern and the like. The flexible part 72 electrically connects the rigid parts 71 that are adjacent to each other among the rigid parts 71. That is, the flexible part 72 electrically connects at least two of the rigid parts 71 to each other among the rigid parts 71. The flexible parts 72 have a band shaped form with a width direction thereof the same as the traveling direction of the yarn Y. When seen from the traveling direction of the yarn Y, the rigid parts 71 and the flexible parts 72 encircle the traveling area R1. The encirclement is not limited to a complete encirclement that completely encircles (360 degrees) the traveling area R1 but can be a partial encirclement that encircles a part of the traveling area R1. In the example shown in the drawings, when seen from the traveling direction of the yarn Y, when a principal surface of the rigid parts 71 faces toward the traveling area R1, the rigid parts 71 surround the traveling area R1 and the flexible parts 72 surround the traveling area R1 in a region between the rigid parts 71.

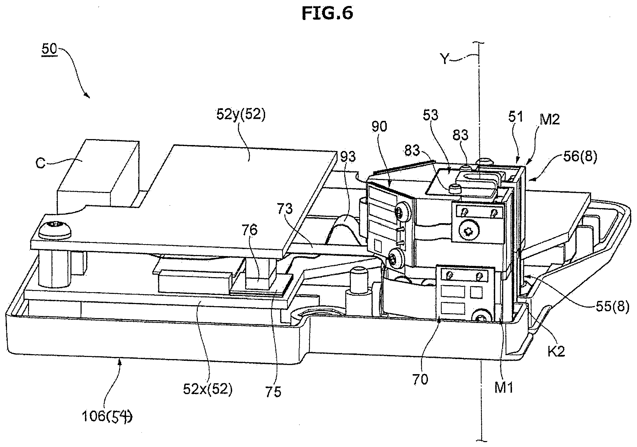

The flexible connecting part 73 electrically connects the light emitting element 57 and the light receiving element 58, which are mounted on the first holder 55, to a first flat substrate 52x (see FIG. 6). One end of the flexible connecting part 73 is electrically connected to a rigid part 71z arranged on the back side surface of the first holder 55. A relay substrate 75 is arranged at other end of the flexible connecting part 73. The relay substrate 75 is provided with a connector part 76. The connector part 76 is electrically connected to a connector part of the first flat substrate 52x. With this arrangement, the first flat substrate 52x inputs signals (electrical signals) to and receives signals from the light emitting element 57 and the light receiving element 58 via the rigid flexible substrate 70.

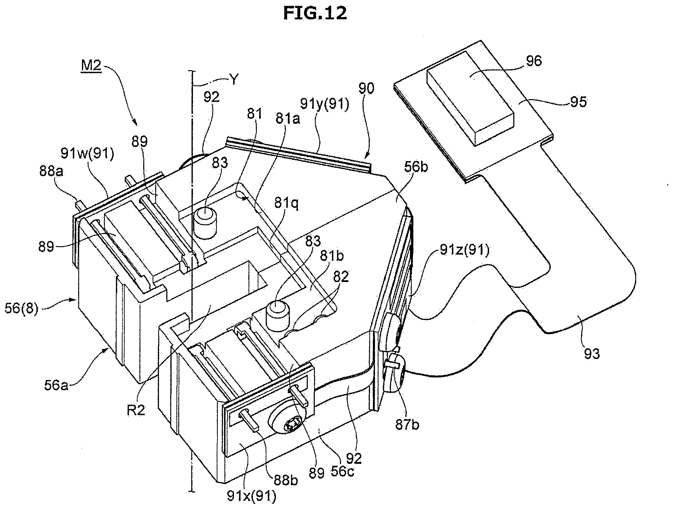

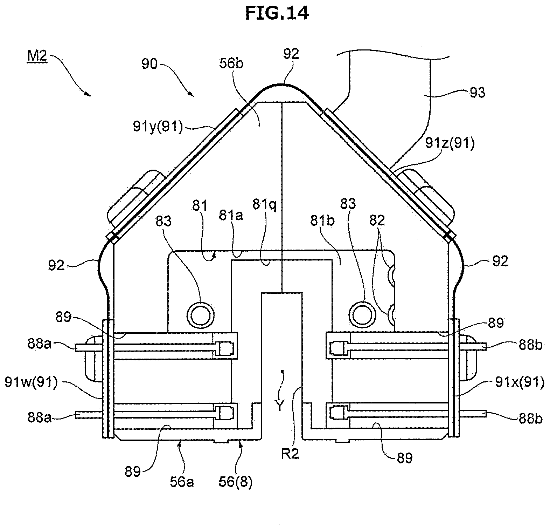

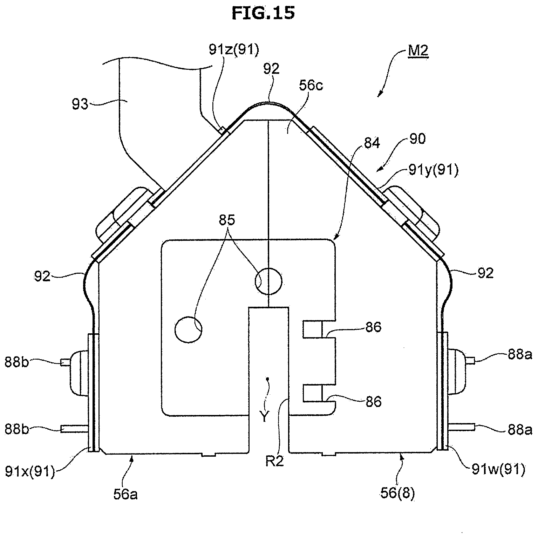

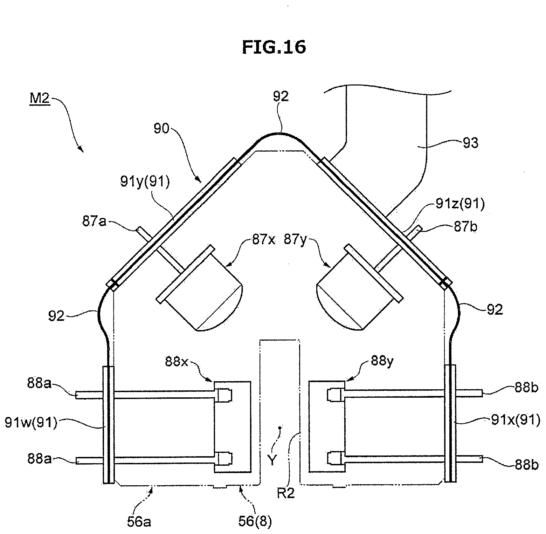

As shown in FIGS. 12 to 16, the second holder 56 constitutes a second detection module M2 through which the traveling yarn Y is passed to detect the state of the yarn Y. The second detection module M2 has at least a foreign substance detection function to detect a foreign substance contained in the traveling yarn Y. The second holder 56 is arranged upstream of the first holder 55. Among the holders 8, the second holder 56 is arranged at one end in the traveling direction of the yarn Y. The second holder 56 includes a body 56a that is made of, for example, resin. The second holder 56 is separable into a left part and a right part (in a left-right direction that is the travelling direction of the yarn Y and a direction that is orthogonal to the front-back direction). Thus, the second holder 56 is constituted by a pair of separable parts that is a combination of the left part and the right part. However, the second holder 56 can be an integral body that cannot be separated into the left part and the right part.

The body 56a of the second holder 56 is provided with a traveling area R2 through which the yarn Y travels. The traveling area R2 is a space that extends along the traveling path of the yarn Y. The traveling area R2 is open on the front side. In different words, the traveling area R2 is a passage through which the traveling yarn Y passes, and it is formed such that it extends from upstream to downstream and has a groove shape that is open on the front side. When seen from the traveling direction of the yarn Y, the traveling area R2 is formed in a U-shape. The traveling area R2 communicates with the traveling area R1 of the first holder 55.

An upstream concave part 81 is formed in a top surface 56b that is an upstream end surface of the body 56a of the second holder 56. The upstream concave part 81 is formed in a rectangular shape in an inner area from an outer margin of the top surface 56b. When seen from the traveling direction of the yarn Y, the upstream concave part 81 is formed on all sides except on the side on which the traveling area R2 is open.

The upstream concave part 81 is a space in which the yarn path guide 53 is arranged. When seen from the traveling direction of the yarn Y, the upstream concave part 81 has a shape that corresponds with the yarn path guide 53. The upstream concave part 81 has a depth that corresponds with a thickness of the yarn path guide 53. A plurality of ribs 82 is arranged on an inner side surface 81a of the upstream concave part 81. The ribs 82 project inward from the side surface 81a so as to bulge. When the yarn path guide 53 is engaged with the upstream concave part 81, the ribs 82 remove a gap (so-called "wobble", also called as "backlash" or "play") between the yarn path guide 53 and the upstream concave part 81. A step 81q that deepens in a rectangle shape is formed around the traveling area R2 in a bottom surface 81b of the upstream concave part 81. The step 81q engages with the later-explained convex part 102 (see FIG. 17) of the yarn path guide 53.

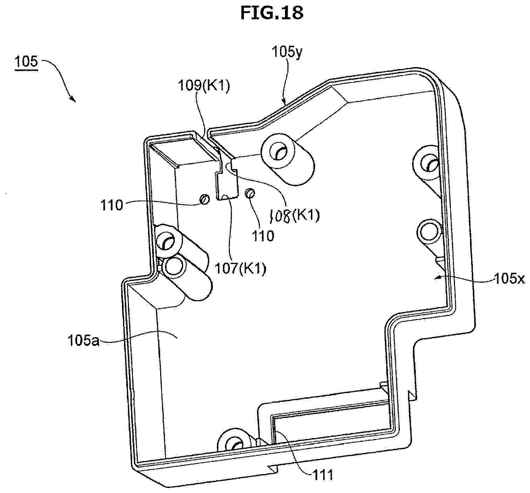

A plurality of upstream convex parts 83 is arranged on the bottom surface 81b of the upstream concave part 81. One upstream convex part 83 is arranged on one side and another upstream convex part 83 is arranged on the other side of the traveling area R2 in the bottom surface 81b. The upstream convex parts 83 protrude further upstream of the top surface 56b. The upstream convex parts 83 engage with later-explained notches 110 (see FIG. 18) of the case 54. When seen from the traveling direction of the yarn Y, the upstream convex parts 83 have a cross-sectional shape that corresponds with the notches 110. The upstream convex parts 83 shown in the drawings have a circular cross-sectional shape. The upstream convex parts 83 constitute the first positioning member that positions the second holder 56 with respect to the case 54.

The downstream convex part 84 is formed in a bottom surface 56c that is a downstream end surface of the body 56a of the second holder 56. The downstream convex part 84 is formed in a rectangular shape in an inner area from an outer margin of the bottom surface 56c. When seen from the traveling direction of the yarn Y, the downstream convex part 84 is formed on all sides except on the side on which the traveling area R2 is open.

The downstream convex part 84 is engaged with and fitted in the upstream concave part 61 of the first holder 55. When seen from the traveling direction of the yarn Y, the downstream convex part 84 has a cross-sectional shape that corresponds with the upstream concave part 61. The downstream convex part 84 has a projecting height that corresponds with a depth of the upstream concave part 61. The downstream convex part 84 is provided with the holes 85 that engage with the upstream convex parts 63 of the first holder 55. When seen from the traveling direction of the yarn Y, the holes 85 have a cross-sectional shape that corresponds with the upstream convex parts 63. The holes 85 shown in the drawings have a circular cross-sectional shape. The downstream convex part 84 is provided with grooves 86 that function as a space to avoid interference with the lead pins 58a of the light receiving element 58 of the first holder 55 when coupling the first and second holders 55 and 56 to each other.

The second holder 56 is a sensor mounting holder for mounting a sensor part for measurement. Light emitting elements 87x and 87y and light receiving elements 88x and 88y that detect the state of the yarn Y traveling in the traveling area R2 are mounted inside the body 56a of the second holder 56. The body 56a supports the light emitting elements 87x and 87y and the light receiving elements 88x and 88y. The light emitting elements 87x and 87y and the light receiving elements 88x and 88y constitute the sensor part for measurement. When seen from the traveling direction of the yarn Y, the light emitting elements 87x and 87y and the light receiving elements 88x and 88y are arranged at different positions around the traveling area R2. The light emitting elements 87x and 87y and the light receiving elements 88x and 88y are arranged such that the optic axis of each of those elements is located on the same plane that is orthogonal to the traveling direction of the yarn Y. An LED and the like, for example, can be used as the light emitting elements 87x and 87y. A photodiode and the like, for example, can be used as the light receiving elements 88x and 88y.

The light emitting element 87x has a pair of lead pins 87a and the light emitting element 87y has a pair of lead pins 87b. Tips of the lead pins 87a and 87b extend away from the traveling area R2. The light receiving element 88x has a pair of lead pins 88a and the light receiving element 88y has a pair of lead pins 88b. Tips of the lead pins 88a and 88b extend away from the traveling area R2 through grooves 89 formed in the top surface 56b.

In the second detection module M2 including the second holder 56 on which the light emitting elements 87x and 87y and the light receiving elements 88x and 88y are mounted, a light is emitted from the light emitting element 87x on the yarn Y traveling in the traveling area R2, a reflected light that is reflected by the yarn Y among the emitted light is received by the light receiving element 88x, and a transmitted light that passes through the yarn Y among the emitted light is received by the light receiving element 88y. On the other hand, a light is emitted from the light emitting element 87y on the yarn Y traveling in the traveling area R2, a reflected light that is reflected by the yarn Y among the emitted light is received by the light receiving element 88y, and a transmitted light that passes through the yarn Y among the emitted light is received by the light receiving element 88x. Then, whether a foreign substance is contained in the yarn Y is detected based on an amount of the reflected light received by the light receiving elements 88x and 88y. In the second detection module M2, the light emitting element 87x and the light emitting element 87y are turned on alternately.

The yarn monitoring device 50 includes a rigid flexible substrate 90. The rigid flexible substrate 90 includes a plurality of rigid parts 91, a plurality of flexible parts 92, and a flexible connecting part 93. The rigid flexible substrate 90 constitutes the second detection module M2.

Rigid material such as glass epoxy is used for the rigid parts 91. A material having high flexibility and bendability such as polyimide is used for the flexible parts 92 and the flexible connecting part 93. If the same material is used for the rigid parts 91, the flexible parts 92, and the flexible connecting part 93, the rigid parts 91 can be made thicker than the flexible parts 92 and the flexible connecting part 93. In short, it is sufficient that the flexibility and bendability of the flexible parts 92 and the flexible connecting part 93 are higher than the flexibility and bendability of the rigid parts 91. It is sufficient that the rigidity of the rigid parts 91 is higher than that of the flexible parts 92 and the flexible connecting part 93.

The rigid part 91 is a rigid substrate provided with a wiring pattern, through holes, and the like. The rigid parts 91 are fixed to side surfaces of the second holder 56. Specifically, in the body 56a of the second holder 56, one rigid part 91 is fixed with one or more screws to each of a pair of first side surfaces that is located across the traveling area R2 and each of a pair of second side surfaces that is inclined to and arranged in the back side of the first side surfaces. Alternatively, the rigid parts 91 can be fixed to the body 56a by welding.

The lead pins 88a of the light receiving element 88x are connected by soldering and the like to a rigid part 91w on the light receiving element 88x side among the rigid parts 91. The lead pins 88b of the light receiving element 88y are connected by soldering and the like to a rigid part 91x on the light receiving element 88y side among the rigid parts 91. The lead pins 87a of the light emitting element 87x are connected by soldering and the like to a rigid part 91y on the light emitting element 87x side among the rigid parts 91. The lead pins 87b of the light emitting element 87y are connected by soldering and the like to a rigid part 91z on the light emitting element 87y side among the rigid parts 91. That is, the light receiving elements 88x and 88y are mounted on the rigid parts 91w and 91x, respectively, and the light emitting elements 87x and 87y are mounted on the rigid parts 91y and 91z, respectively. The rigid parts 91 have a plate shaped form that extends along the traveling direction of the yarn Y.

The flexible part 92 is a flexible substrate provided with a wiring pattern and the like. The flexible part 92 electrically connects the rigid parts 91 that are adjacent to each other among the rigid parts 91. That is, the flexible part 92 electrically connects at least two of the rigid parts 91 to each other among the rigid parts 91. The flexible parts 92 have a band shaped form with a width direction thereof the same as the traveling direction of the yarn Y. When seen from the traveling direction of the yarn Y, the rigid parts 91 and the flexible parts 92 encircle the traveling area R2. The encirclement is not limited to a complete encirclement that completely encircles (360 degrees) the traveling area R2 but can be a partial encirclement that encircles a part of the traveling area R2. In the example shown in the drawings, when seen from the traveling direction of the yarn Y, when a principal surface of the rigid parts 91 faces toward the traveling area R2, the rigid parts 91 surround the traveling area R2 and the flexible parts 92 surround the traveling area R2 in a region between the rigid parts 91.

The flexible connecting part 93 electrically connects the light emitting elements 87x and 87y and the light receiving elements 88x and 88y, which are mounted on the second holder 56, to a second flat substrate 52y (see FIG. 6). One end of the flexible connecting part 93 is electrically connected to the rigid part 91z arranged on the back side surface of the second holder 56. A relay substrate 95 is arranged at other end of the flexible connecting part 93. The relay substrate 95 is provided with a connector part 96. The connector part 96 is electrically connected to a connector part of the second flat substrate 52y. With this arrangement, the second flat substrate 52y inputs signals to and receives signals from the light emitting elements 87x and 87y and the light receiving elements 88x and 88y via the rigid flexible substrate 90.

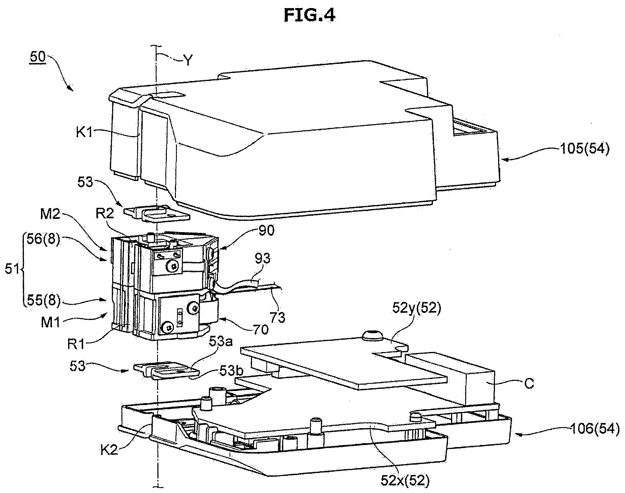

As shown in FIGS. 4 to 6, the flat substrates 52 include the first and second flat substrates 52x and 52y. The first and second flat substrates 52x and 52y are flat control boards. The first flat substrate 52x processes a signal to be input into the light emitting element 57 that is mounted on the first holder 55. The first flat substrate 52x processes a signal received from the light receiving element 58 that is mounted on the first holder 55. The first flat substrate 52x is electrically connected to the rigid part 71z of the first holder 55 via the flexible connecting part 73.

The second flat substrate 52y processes a signal to be input into the light emitting elements 87x and 87y that are mounted on the second holder 56. The second flat substrate 52y processes a signal received from the light receiving elements 88x and 88y that are mounted on the second holder 56. The second flat substrate 52y is electrically connected to the rigid part 91z of the second holder 56 via the flexible connecting part 93. The first and second flat substrates 52x and 52y correspond to the first and second holders 55 and 56. The number of the flat substrates 52 is equal to the number of the holders 8. In different words, the flat substrates 52 are provided in the number that is equal to the number of the holders 8.

Integrated circuits containing electronic parts (processors and the like) for signal processing are mounted on each of the first and second flat substrates 52x and 52y. The first and second flat substrates 52x and 52y are arranged and accommodated in the case 54 so that mounting surfaces thereof are orthogonal to the traveling direction of the yarn Y. The first and second flat substrates 52x and 52y are piled up one above the other in the traveling direction of the yarn Y. In the present embodiment, the first flat substrate 52x is fixed with one or more screws to a lower casing 106 (explained later) of the case 54. Alternatively, the first flat substrate 52x can be fixed to the lower casing 106 by welding. The second flat substrate 52y is fixed to and connected via a connector to the first flat substrate 52x in an overlapping manner but with a gap between the first flat substrate 52x.

As shown in FIGS. 4, 17A, and 17B, the yarn path guides 53 regulate the traveling path of the yarn Y and guide the traveling yarn Y. One yarn path guide 53 is arranged inside the case 54 on each of upstream and downstream sides of the holder coupler 51. Specifically, a first yarn path guide 53 is arranged (in the downstream concave part 64) in the traveling direction of the yarn Y between the first holder 55 and the case 54. A second yarn path guide 53 is arranged (in the upstream concave part 81) in the traveling direction of the yarn Y between the second holder 56 and the case 54. The first yarn path guide 53 is arranged such that the upstream and the downstream sides are reversed with respect to the second yarn path guide 53 (that is, in the vertical direction, the up-down orientation of the second yarn path guide 53 is opposite of the first yarn path guide 53).

The yarn path guide 53 is formed of a material (e.g., ceramic, titanium, and the like) having resistance to wear. When seen from the traveling direction of the yarn Y, the yarn path guides 53 have a shape of a rectangular plate that corresponds with a shape of the downstream concave part 64 of the first holder 55 and a shape of the upstream concave part 81 of the second holder 56. When seen from the traveling direction of the yarn Y, a groove 101 having a U-shape is formed in the yarn path guide 53. The inner side of the groove 101 functions as a yarn guiding part that guides the yarn Y to the traveling areas R1 and R2 of the first and second holders 55 and 56.

The convex part 102 is arranged on a surface 53a, which faces toward the holder coupler 51, of the yarn path guide 53. The convex part 102 is arranged around the groove 101 in the surface 53a and continuous with the groove 101. The convex part 102 of the yarn path guide 53 arranged between the first holder 55 and the case 54 engages with the step 64q formed in the downstream concave part 64 of the first holder 55. The convex part 102 of the yarn path guide 53 arranged between the second holder 56 and the case 54 engages with the step 81q formed in the upstream concave part 81 of the second holder 56. The convex parts 102 have projecting heights lower than the depths of the steps 64q and 81q. A protruding part 103 is arranged on a surface 53b, which faces toward the case 54, of the yarn path guide 53. This protruding part 103 functions to secure a gap between the yarn path guide 53 and the case 54. The protruding part 103 is arranged around the groove 101 in the surface 53b and continuous with the groove 101.

The yarn path guide 53 is provided with a plurality of (in the present embodiment, two) through-holes 104. The through-holes 104 of the yarn path guide 53 arranged between the first holder 55 and the case 54 engage with the downstream convex parts 66 of the first holder 55. The through-holes 104 of the yarn path guide 53 arranged between the second holder 56 and the case 54 engage with the upstream convex parts 83 of the second holder 56. When seen from the traveling direction of the yarn Y, the through-holes 104 have a cross-sectional shape that corresponds with the downstream convex parts 66 and the upstream convex parts 83. The through-holes 104 shown in the drawings have a circular cross-sectional shape. The through-holes 104 constitute a third positioning member that positions the yarn path guide 53 with respect to the case 54 and the holder coupler 51.

As shown in FIGS. 3 and 4, the case 54 constitutes an outer part of the yarn monitoring device 50. The case 54 is constituted by an upper casing (a first casing section) 105 arranged upstream and the lower casing (a second casing section) 106 arranged downstream. The upper casing 105 and the lower casing 106 can be coupled to each other with one or more screws. The holder coupler 51, the first and second flat substrates 52x and 52y, and the yarn path guides 53 are accommodated inside the case 54.

As shown in FIGS. 4 and 18, a cutout portion K1 is formed in the upper casing 105, along the traveling path of the yarn Y, corresponding to the traveling areas R1 and R2 of the first and second holders 55 and 56. Specifically, an opening 107 is formed in a front side of an upper wall 105x on the upstream in the upper casing 105. The opening 107 has a rectangular cross section and is bored through the upper wall 105x. A groove 108 that is continuous with the opening 107 is formed in the upper wall 105x. The groove 108 leads to a front edge of the upper wall 105x from the opening 107. A groove 109 is formed in a front wall 105y on the front side in the upper casing 105. The groove 109 is continuous with the groove 108 of the upper wall 105x. The groove 109 extends from an upstream edge to a downstream edge of the front wall 105y. The cutout portion K1, constituted by the opening 107 and the grooves 108 and 109, is a space in which the yarn Y can travel in the traveling areas R1 and R2 and can be advanced and retreated with respect to the traveling areas R1 and R2, and communicates with the traveling areas R1 and R2.

The notches 110 are formed in an inner surface 105a of the upper wall 105x of the upper casing 105. The notches 110 engage with the upstream convex parts 83 of the second holder 56. As shown in the drawing, a pair of the notches 110 is arranged near the opening 107 of the inner surface 105a but on either sides of the opening 107. When seen from the traveling direction of the yarn Y, the notches 110 have a cross-sectional shape that corresponds with the upstream convex parts 83. In the present embodiment, the notches 110 have a circular cross-sectional shape. The notches 110 constitute a second positioning member that positions the second holder 56 with respect to the case 54. A through-hole 111 having a rectangular cross section is formed in the upper wall 105x as an opening for exposing a connector C of the first flat substrate 52x to the outside.

As shown in FIGS. 4 and 19, a cutout portion K2 is formed in the lower casing 106, along the traveling path of the yarn Y, corresponding to the traveling area R2 of the second holder 56. Specifically, an opening 112 is formed in a front side of a bottom wall 106x on the downstream in the lower casing 106. The opening 112 has a rectangular cross section and is bored through the bottom wall 106x. A groove 113 that is continuous with the opening 112 is formed in the bottom wall 106x. The groove 113 leads to a front edge of the bottom wall 106x from the opening 112. A groove 114 is formed in a front wall 106y on the front side in the lower casing 106. The groove 114 is continuous with the groove 113 of the bottom wall 106x. Moreover, the groove 114 is continuous with the groove 109 of the upper casing 105 when the upper casing 105 is coupled to the lower casing 106. The groove 114 extends from an upstream edge to a downstream edge of the front wall 106y. The cutout portion K2, constituted by the opening 112 and the grooves 113 and 114, is a space in which the yarn Y can travel in the traveling area R2 and can be advanced and retreated with respect to the traveling area R2, and communicates with the traveling area R2.

The notches 115 are formed in an inner surface 106a of the bottom wall 106x of the lower casing 106. The notches 115 engage with the downstream convex parts 66 of the first holder 55. As shown in the drawing, a pair of the notches 115 is arranged near the opening 112 of the inner surface 106a but on either sides of the opening 112. When seen from the traveling direction of the yarn Y, the notches 115 have a cross-sectional shape that corresponds with the downstream convex parts 66. In the present embodiment, the notches 115 have a circular cross-sectional shape. The notches 115 constitute the second positioning member that positions the first holder 55 with respect to the case 54.

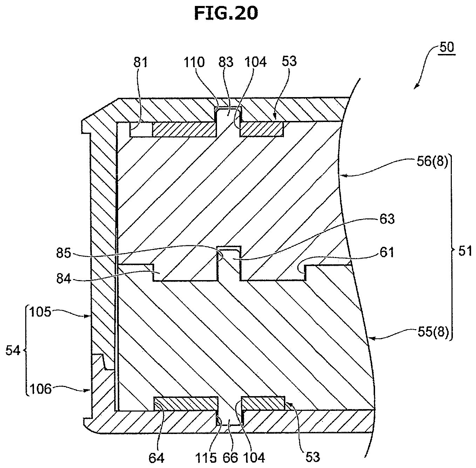

FIG. 20 is a sectional view of the yarn monitoring device 50 along a plane that passes through the downstream convex parts 66 of the first holder 55 and the upstream convex parts 83 of the second holder 56 and that is parallel to the front-back direction and to the traveling direction of the yarn Y. As shown in FIGS. 4 to 6 and FIG. 20, in the yarn monitoring device 50 having the above-explained configuration, the first and second holders 55 and 56 are piled up one above the other in the traveling direction of the yarn Y so that the traveling areas R1 and R2 communicate with each other. When doing so, the downstream convex part 84 of the second holder 56 is inserted into the upstream concave part 61 of the first holder 55 and the upstream convex parts 63 of the first holder 55 are inserted into the holes 85 of the second holder 56. With this arrangement, the first and second holders 55 and 56 are detachably coupled to each other because of the engagement of the concave and convex parts leading to formation of the holder coupler 51.

When the upper casing 105 and the lower casing 106 are coupled to each other, the holder coupler 51, the first and second flat substrates 52x and 52y, and the pair of the yarn path guides 53 are accommodated in the case 54. The holder coupler 51 is arranged in the front side of the case 54 so that the traveling areas R1 and R2 communicate with the cutout portions K1 and K2. The first and second flat substrates 52x and 52y are arranged in a piled up manner in the case 54, in an area other than where the holder coupler 51 is arranged, such that the mounting surfaces thereof are orthogonal to the traveling direction of the yarn Y. The first yarn path guide 53 is arranged in the downstream concave part 64 of the first holder 55 and is intervened between the first holder 55 and the lower casing 106. The second yarn path guide 53 is arranged in the upstream concave part 81 of the second holder 56 and is intervened between the second holder 56 and the upper casing 105.

As shown in FIG. 20, in the state in which the lower casing 106 has been coupled to the upper casing 105 (hereinafter, simply "coupled state"), the first and second holders 55 and 56 that have been coupled to each other are held between the upper casing 105 and the lower casing 106 in the traveling direction of the yarn Y and are pinched in the traveling direction of the yarn Y. In this state, the downstream convex parts 66 of the first holder 55 are inserted into the notches 115 of the lower casing 106 whereby the first holder 55 is positioned, and the upstream convex parts 83 of the second holder 56 are inserted into the notches 110 of the upper casing 105 whereby the second holder 56 is positioned. That is, the first and second holders 55 and 56 are positioned in the case 54 by being held in the traveling direction and by the downstream convex parts 66 engaging with the notches 115 and the upstream convex parts 83 engaging with the notches 110.

Furthermore, in the coupled state, the downstream convex parts 66 of the first holder 55 are inserted into the through-holes 104 in the yarn path guide 53 located between the first holder 55 and the lower casing 106. With this arrangement, the yarn path guide 53 is also positioned with respect to the first holder 55 that has been positioned in the case 54. Similarly, in the coupled state, the upstream convex parts 83 of the second holder 56 are inserted into the through-holes 104 in the yarn path guide 53 located between the second holder 56 and the upper casing 105. With this arrangement, the yarn path guide 53 is also positioned with respect to the second holder 56 that has been positioned in the case 54. That is, the first and second holders 55 and 56, the yarn path guides 53, and the case 54 are positioned by being held in the traveling direction of the yarn Y, and by the downstream convex parts 66 that are engaged with the notches 115 also engaging with the through-holes 104 and the upstream convex parts 83 that are engaged with the notches 110 also engaging with the through-holes 104.

In the yarn monitoring device 50, the ribs 62 arranged in the upstream concave part 61 of the first holder 55 contact the side surfaces of the downstream convex part 84 of the second holder 56 and are deformed whereby the downstream convex part 84 is fitted within and engages with the upstream concave part 61. With this arrangement, wobble, which may be produced between the upstream concave part 61 and the downstream convex part 84, can be removed. In the yarn monitoring device 50, the ribs 65 arranged in the downstream concave part 64 of the first holder 55 contact the side surfaces of the yarn path guide 53 and are deformed whereby the yarn path guide 53 is fitted within and engages with the downstream concave part 64. With this arrangement, wobble, which may be produced between the downstream concave part 64 and the yarn path guide 53, can be removed. In the yarn monitoring device 50, the ribs 82 arranged in the upstream concave part 81 of the second holder 56 contact the side surfaces of the yarn path guide 53 and are deformed whereby the yarn path guide 53 is fitted within and engages with the upstream concave part 81. With this arrangement, wobble, which may be produced between the upstream concave part 81 and the yarn path guide 53, can be removed.

In this manner, in the yarn monitoring device 50, the first and second flat substrates 52x and 52y are arranged in the first and second holders 55 and 56, respectively. Specifically, the first flat substrate 52x is arranged corresponding to the sensor part for measurement (the light emitting element 57 and the light receiving element 58) of the first holder 55, and the second flat substrate 52y is arranged corresponding to the sensor part for measurement (the light emitting elements 87x and 87y and the light receiving elements 88x and 88y) of the second holder 56. Moreover, the sensor part for measurement of the first holder 55 is connected to the first flat substrate 52x via the flexible connecting part 73, and the sensor part for measurement of the second holder 56 is connected to the second flat substrate 52y via the flexible connecting part 93.

Accordingly, only the holder 8 (the first holder 55 and / or the second holder 56), or only the flat substrate 52 (the first flat substrate 52x and / or the second flat substrate 52y) can be easily replaced or removed. Therefore, a requirement to replace the holder 8 and the flat substrate 52 together as one unit can be reduced. Moreover, the holder 8 to be used can be appropriately changed, and, the arrangement of the flat substrate 52 can be appropriately changed depending on the used holder 8. As a result, it is possible to easily cope with the specification change of the holder 8.

That is, according to the yarn monitoring device 50, an arrangement state of the flat substrates 52 can be appropriately changed depending on an arrangement state of the holders 8. In different words, the flat substrates 52 can be arranged appropriately to support the holders 8. Particularly, when using a dummy holder, which does not have a sensor part for measurement, as the first holder 55 or the second holder 56, omission of the first flat substrate 52x or the second flat substrate 52y can be easily realized.

In the yarn monitoring device 50, the first holder 55 is detachably coupled to the second holder 56 (in other words, the second holder 56 is detachably coupled to the first holder 55). As a result, the first holder 55 or the second holder 56 can be changed easily.

In the yarn monitoring device 50, the first holder 55 includes the downstream convex parts 66 as the first positioning member, and the second holder 56 includes the upstream convex parts 83 as the first positioning member. The case 54 is provided with the notches 110 and 115 as the second positioning member. In the coupled state, the first and second holders 55 and 56 are held between the upper casing 105 and the lower casing 106 in the predetermined direction, which is the traveling direction of the yarn Y, and are positioned in the case 54 by the first positioning member and the second positioning member. Accordingly, the first and second holders 55 and 56 can be held together, and the first and second holders 55 and 56 can be positioned with respect to the case 54. Therefore, the first and second holders 55 and 56 can be arranged with high positioning accuracy and good workability.

In the yarn monitoring device 50, the yarn path guide 53 is intervened between each of the first and second holders 55 and 56 and the case 54. The yarn path guide 53 includes the through-holes 104 as the third positioning member. The first and second holders 55 and 56, the yarn path guides 53, and the case 54 are positioned by the first to third positioning members. According to such a configuration, the yarn path guides 53 are also held in the predetermined direction, which is the traveling direction of the yarn Y, and the yarn path guides 53 can be positioned with respect to the first and second holders 55 and 56 and the case 54. Therefore, the yarn path guides 53 can be arranged with high positioning accuracy. Moreover, because no adhesive and the like is used in the positioning of the yarn path guides 53, the workability can be improved.

In the yarn monitoring device 50, the first positioning member is the downstream convex parts 66, the second positioning member is the notches 115 in which the downstream convex parts 66 engage, and the third positioning member is the through-holes 104 in which the downstream convex parts 66 are inserted. Moreover, the first positioning member is the upstream convex parts 83, the second positioning member is the notches 110 in which the upstream convex parts 83 engage, and the third positioning member is the through-holes 104 in which the upstream convex parts 83 are inserted. According to such a configuration, the positioning of the first and second holders 55 and 56, the yarn path guides 53, and the case 54 can be implemented with a simple configuration and efficiently.

In the yarn monitoring device 50, the first holder 55 is coupled to the second holder 56 with the engagement of the concave and convex parts (in other words, the second holder 56 is coupled to the first holder 55 with the engagement of the concave and convex parts). With this arrangement, the need to perform an adhesion work when coupling the first holder 55 to the second holder 56 (in other words, when coupling the second holder 56 to the first holder 55) can be eliminated.

In the yarn monitoring device 50, the rigid parts 71 and the flexible parts 72 of the rigid flexible substrate 70 are arranged surrounding the traveling area R1 when seen from the traveling direction of the yarn Y. Moreover, the rigid parts 91 and the flexible parts 92 of the rigid flexible substrate 90 are arranged surrounding the traveling area R2 when seen from the traveling direction of the yarn Y. As a result, the rigid flexible substrates 70 and 90 can be arranged in a compact manner.

In the yarn monitoring device 50, the first detection module M1 has at least the yarn thickness detection function, and the second detection module M2 has at least the foreign substance detection function. As a result, according to the yarn monitoring device 50, the thickness of the yarn Y and whether a foreign substance is contained in the yarn Y can be monitored.

The spinning machine 1 is the yarn winding machine that forms the package P by winding the yarn Y, and the spinning machine 1 includes the yarn monitoring device 50. According to the spinning machine 1, in the yarn monitoring device 50, it is possible to easily cope with the specification change of the holder 8.

In the present embodiment, the flat substrates 52 are provided in the number that is equal to the number of the holders 8; however, the configuration is not limited to this. That is, one or more flat substrates 52 can be provided for each of the holders 8. That is, one flat substrate 52 can be provided for each of the holders 8, or a plurality of the flat substrates 52 can be provided for each of the holders 8, for example, by juxtaposing on one plane. The flat substrate 52 processes a signal received from the sensor part for measurement mounted on the corresponding holder 8. In this configuration, the sensor part for measurement in each of the holders 8 is electrically connected to the corresponding one or more flat substrates 52 via the flexible connecting part.

### Second Embodiment

A yarn monitoring device according to a second embodiment is explained below. In the following, differences compared to the first embodiment will be explained and redundant explanation will be omitted.

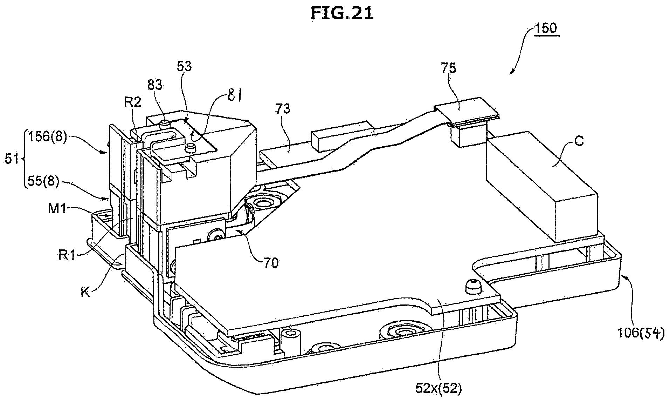

As shown in FIG. 21, a yarn monitoring device 150 according to the second embodiment includes a dummy holder (a holder) 156 instead of the second holder 56. The dummy holder 156 is a sensor not-mounted holder on which no sensor part for measurement is mounted. The dummy holder 156 does not include the light emitting elements 87x and 87y, the light receiving elements 88x and 88y, and the rigid flexible substrate 90. The dummy holder 156 shown in the drawing has the same configuration as the second holder 56 except that the light emitting elements 87x and 87y, the light receiving elements 88x and 88y, and the rigid flexible substrate 90 are not provided.

The yarn monitoring device 150 includes only the first flat substrate 52x and does not include the second flat substrate 52y. That is, the flat substrates 52 are provided in the number (in the present embodiment, one) that is equal to the number of the first holder 55 that is the sensor mounting holder. In the present embodiment, a substrate that is not flat plate shaped can be used instead of the flat substrate 52. The flat substrates 52 are provided in the number that is equal to the number of the sensor mounting holders; however, the configuration is not limited to this. One or more flat substrates 52 can be provided for each of the sensor mounting holders. That is, one flat substrate 52 can be provided for each of the sensor mounting holders, or a plurality of the flat substrates 52 can be provided for each of the sensor mounting holders, for example, by juxtaposing on one plane. The flat substrate 52 processes a signal received from the sensor part for measurement mounted on the corresponding sensor mounting holder.

In this manner, in the yarn monitoring device 150, the first holder 55, which is the sensor mounting holder, is used for a part of the holders 8 among the holder 8, and the dummy holder 156, which is the sensor not-mounted holder, is used for the remaining part of the holders 8. As a result, when making a specification change from a specification in which the first and second holders 55 and 56 are provided (a specification in which all of the holders 8 are the sensor mounting holders), there is no need to use a case having a different shape because the specification change can be handled by using the case 54 having the same configuration. As a result, it is possible to easily cope with the specification change of the holder 8.

The embodiments of the present invention are explained above. The present invention, however, is not limited to the above embodiments.

In the above embodiments, the first holder 55 is positioned directly. That is, the first holder 55 is positioned directly by the case 54 as the downstream convex parts 66 of the first holder 55 directly engage with the notches 115 of the case 54. However, the first holder 55 can be positioned indirectly. Similarly, the second holder 56 is positioned directly. That is, the second holder 56 is positioned directly by the case 54 as the upstream convex parts 83 of the second holder 56 directly engage with the notches 110 of the case 54. However, the second holder 56 can be positioned indirectly. Performing the positioning indirectly means performing positioning by the first positioning member of the holder and the second positioning member of the case via another member. For example, performing the positioning indirectly means, performing the positioning by engaging the holder 8 and the yarn path guide 53 and engaging the yarn path guide 53 and the case 54.

The above embodiments include two holders, i.e., the first and second holders 55 and 56; however, it is allowable to include three or more holders. In the above embodiments, as the third positioning member, the through-holes 104 are provided in the yarn path guide 53; however, instead of the through-holes 104, a cutout portion that engages with the first positioning member can be provided in the yarn path guide 53. The first positioning member can be provided to each of the two holders 8 located on both the sides in the direction in which the holders 8 are arranged, or can be provided to one of those two holders 8.

In the above embodiments, the sensor part for measurement that detects the state of the yarn Y is not limited to an optical sensor part for measurement. For example, the sensor part for measurement can be an electrostatic capacitive sensor part for measurement such as a pair of opposing electrodes and the like is arranged across the traveling area of the yarn Y. The sensor part for measurement is not limited to a leaded part having lead pins as connectors. For example, the sensor part for measurement can be a surface mount device having electrode pads and the like as the connectors.

In the above embodiments, each of the first detection module M1 and the second detection module M2 can have, for example, a function to detect a traveling speed of the yarn Y, a function to detect a traveling length of the yarn Y, and the like, instead of or in addition to the yarn thickness detection function and the foreign substance detection function. It is sufficient that the first detection module M1 and / or the second detection module M2 have a function to detect at least one state of the traveling yarn Y. The yarn thickness detection function of the first detection module M1 includes not only optical detection of the apparent thickness of the yarn Y but also the mass detection of the yarn Y by using an electrostatic capacitance.

In the above embodiments, the notches 115 and 110 are formed in the case 54, and the convex parts (the downstream convex parts 66 and the upstream convex parts 83) that engage with the notches 115 and 100 are formed in the holders 8; however, a positioning structure for positioning the case and the holders is not limited to this. For example, contrary to the above embodiments, the convex parts can be formed in the case and the notches that engage with those convex parts can be formed in the holder.

In the above embodiments, among the holders 8, the adjacent holders 8 are coupled to each other with the engagement of the concave and convex parts; however, instead of or in addition to this, the adjacent holders 8 can be coupled to each other by adhesion. Similarly, the yarn path guide 53 and the holder 8 can be coupled to each other by adhesion. The term "couple / coupled" means a state in which the two objects are engaged with each other via some kind of a positional relationship holding means. In the above embodiments, the first positioning member and the second positioning member collaborate (that is, the first positioning member and the second positioning member engage with each other) to position the holder 8 with respect to the case 54. However, it is allowable that the first positioning member and the second positioning member do not collaborate, but each of these can be separately position the holder 8 with respect to the case 54.

The air spinning device 7, to prevent the twist of the fiber bundle F from being conveyed to the upstream of the air spinning device 7, can further include a needle held by the fiber guiding section and projecting in the spinning chamber. The air spinning device 7 can prevent the twist of the fiber bundle F from being conveyed to the upstream of the air spinning device 7, instead of using the needle, by using a downstream end portion of the fiber guiding section. Furthermore, in the air spinning device 7, instead of the above configuration, a pair of air jet nozzles that twist the fiber bundle F in mutually opposite directions can be arranged.

In the spinning unit 2, the yarn accumulating device 11 is used to pull the yarn Y from the air spinning device 7; however, a delivery roller and a nip roller can be used to pull the yarn Y from the air spinning device 7. In a configuration in which the yarn Y is pulled from the air spinning device 7 with the delivery roller and the nip roller, instead of the yarn accumulating device 11, a slack tube that absorbs the slack of the yarn Y by using a suction airflow, a mechanical compensator, and the like can be provided.

In the spinning machine 1, various devices are arranged such that the yarn Y supplied from the upper side is wound on the lower side in a height direction of the machine frame. However, those devices can be arranged such that the yarn Y supplied from the lower side is wound on the upper side.

In the spinning machine 1, the traversing guide 23 and at least one bottom roller of the drafting device 6 are driven by a power source arranged in the second-end frame 5 (i.e., the power source is shared among a plurality of the spinning units 2). However, each component (e.g., the drafting device 6, the air spinning device 7, the winding device 13, and the like) of each spinning unit 2 can be driven independently in each spinning unit 2.

The tension sensor 9 can be arranged upstream of the yarn monitoring device 50 in the traveling direction of the yarn Y. One unit controller 10 can be arranged for each spinning unit 2. Moreover, in the spinning unit 2, the waxing device 12, the tension sensor 9, and the yarn monitoring device 50 can be omitted.

In FIG. 1, the spinning machine 1 is shown to wind the yarn Y into a cheese-shaped package P; however, it is possible to wind the yarn Y into a cone-shaped package P. The yarn Y may slacken when traversing the yarn Y for winding into the cone-shaped package P; however, such slack can be absorbed by the yarn accumulating device 11. The material and shape of various components are not limited to those mentioned above, and it is possible to adopt various materials and shapes. The yarn winding machine according to the present invention is not limited to the spinning machine 1 but can be an automatic winder that is constituted by, for example, a plurality of winder units.

In the above explanation, the meaning of "a plurality of" also includes "a predetermined number of'. Although the invention has been explained with respect to specific embodiments for a complete and clear disclosure, the appended claims are not to be thus limited but are to be construed as embodying all modifications and alternative constructions that may occur to one skilled in the art that fall within the scope of the claims.

## Claims

1. A yarn monitoring device (50) comprising:
a plurality of holders (8, 55, 56) that are piled up one above the other along a predetermined direction and through which a traveling yarn (Y) passes;
at least one substrate (52, 52x, 52y); and
a case (54) that accommodates the holders (8, 55, 56) and the at least one substrate (52, 52x, 52y), wherein
a sensor part for measurement (57, 58, 87x, 87y, 88x, 88y) that is configured to measure a state of the traveling yarn (Y) is mounted on at least one of the holders (55, 56),
the at least one substrate (52, 52x, 52y) is associated with the holder (55, 56) on which the sensor part for measurement (57, 58, 87x, 87y, 88x, 88y) is mounted, and the at least one substrate (52x, 52y) is configured to process a signal received from the sensor part for measurement (57, 58, 87x, 87y, 88x, 88y) mounted on the holder (55, 56),
the sensor part for measurement (57, 58, 87x, 87y, 88x, 88y) is electrically connected to the at least one substrate (52, 52x, 52y) via a flexible connecting part (73, 93), and
two adjacent holders (55, 56; 55, 156) among the holders (8, 55, 56; 8, 55, 156) are detachably coupled to each other with engagement of concave and convex parts.

2. The yarn monitoring device (50) as claimed in Claim 1,
wherein the at least one substrate (52, 52x, 52y) is one of a plurality of substrates (52, 52x, 52y),
wherein the case (54) accommodates the holders (8, 55, 56) and the substrates (52, 52x, 52y),
wherein a sensor part for measurement (57, 58, 87x, 87y, 88x, 88y) that is configured to measure a state of the traveling yarn (Y) is mounted on each of the holders (55, 56),
wherein at least one of the plurality of substrates (52, 52x, 52y) is associated with each of the holders (55, 56) and each of the substrates (52x, 52y) is configured to process a signal received from the sensor part for measurement (57, 58, 87x, 87y, 88x, 88y) mounted on the associated holder (55, 56), and
the sensor part for measurement (57, 58, 87x, 87y, 88x, 88y) in each of the holders (55, 56) is electrically connected to the associated at least one substrate (52, 52x, 52y) via the flexible connecting part (73, 93)

3. The yarn monitoring device (150) as claimed in claim 1, wherein:
a subset of the holders (8, 55, 156) is a sensor mounting holder (55) on which a sensor part for measurement (57, 58, 87x, 87y, 88x, 88y) that is configured to measure a state of the traveling yarn (Y) is mounted,
a remaining subset of the holders (8, 55, 156) is a sensor not-mounted holder (156) on which no sensor part for measurement is mounted, and
at least one substrate (52, 52x, 52y) is associated with each of the sensor mounting holders (55) and each substrate (52x, 52y) is configured to process a signal received from the sensor part for measurement (57, 58, 87x, 87y, 88x, 88y) mounted on the associated sensor mounting holder (55).

4. The yarn monitoring device (50; 150) as claimed in any one of Claims 1 to 3, wherein
the case (54) includes at least a first casing section (105) and a second casing section (106) couplable to the first casing section (105), and
the case (54) accommodates the holders (8, 55, 56; 8, 55, 156) in a coupled state in which the second casing section (106) is coupled to the first casing section (105).

5. The yarn monitoring device (50; 150) as claimed in claim 4, wherein
an end holder (55, 56; 55, 156) among the holders (8, 55, 56; 8, 55, 156) arranged at an end in the predetermined direction includes a first positioning member (66, 83) that is configured to position the end holder (55, 56; 55, 156) with respect to the case (54),
the case (54) includes a second positioning member (110, 115) that is configured to directly or indirectly position the end holder (55, 56; 55, 156) with respect to the case (54), and
in the coupled state, the holders (8, 55, 56; 8, 55, 156) are held by the first casing section (105) and the second casing section (106) in a direction parallel to the predetermined direction and the holders (8, 55, 56; 8, 55, 156) are positioned in the case (54) by the first positioning member (66, 83) and the second positioning member (110, 115).

6. The yarn monitoring device (50; 150) as claimed in claim 5, further comprising a plurality of yarn path guides (53) that guides the traveling yarn (Y), wherein
at least one end yarn path guide (53) arranged at an end in the predetermined direction among the yarn path guides (53) is arranged between the end holders (55, 56; 55, 156) and the case (54),
the yarn path guides (53) include a third positioning member (104) that is configured to position the yarn path guides (53) with respect to the case (54) and the holders (55, 56; 55, 156),
the holders (8, 55, 56; 8, 55, 156), the case (54), and the yarn path guides (53) are positioned by the first positioning member (66, 83), the second positioning member (110, 115), and the third positioning member (104).

7. The yarn monitoring device (50; 150) as claimed in claim 6, wherein
one of the first positioning member (66, 83) and the second positioning member (110, 115) is a convex part,
the other of the first positioning member (66, 83) and the second positioning member (110, 115) is a notch in which the convex part engages, and
the third positioning member (104) is a through-hole for inserting the convex part, or a cutout portion in which the convex part engages.

8. The yarn monitoring device (50; 150) as claimed in any one of Claims 1 to 7, further comprising a rigid flexible substrate (70, 90), which is different from the substrates (52, 52x, 52y), including a plurality of rigid parts (71, 71x, 71y, 71z, 91, 91x, 91y, 91z, 91w) and a plurality of flexible parts (72, 92), wherein
the sensor part for measurement (57, 58, 87x, 87y, 88x, 88y) is mounted on each of the rigid parts (71, 71x, 71y, 71z, 91, 91x, 91y, 91z, 91w),
the flexible parts (72, 92) electrically connect at least two of the rigid parts (71, 71x, 71y, 71z, 91, 91x, 91y, 91z, 91w) to each other, and
the rigid parts (71, 71x, 71y, 71z, 91, 91x, 91y, 91z, 91w) and the flexible parts (72, 92) are arranged surrounding a traveling area of the yarn (Y) when seen from the predetermined direction.

9. The yarn monitoring device (50; 150) as claimed in any one of Claims 1 to 8, wherein
the holders (8, 55, 56; 8, 55, 156) include
a first holder (55) that constitutes a first detection module (M1) having at least a function to detect a thickness of the yarn (Y), and
a second holder (56) that constitutes a second detection module (M2) having at least a function to detect a foreign substance contained in the yarn (Y).

10. The yarn monitoring device (50; 150) as claimed in any one of Claims 1 to 9, wherein the substrate (52, 52x, 52y) is a flat substrate.

11. A yarn winding machine (1) that is configured to wind a yarn (Y) to form a package (P), comprising the yarn monitoring device (50; 150) as claimed in any one of Claims 1 to 9, wherein the predetermined direction is a traveling direction of the yarn (Y).

## Patentansprüche

1. Eine Garnüberwachungsvorrichtung (50), die folgende Merkmale aufweist:
eine Mehrzahl von Haltern (8, 55, 56), die entlang einer vorbestimmten Richtung übereinander gestapelt sind und durch die ein sich bewegendes Garn (Y) verläuft;
zumindest ein Substrat (52, 52x, 52y) und
ein Gehäuse (54), das die Halter (8, 55, 56) und das zumindest eine Substrat (52, 52x, 52y) aufnimmt, wobei
ein Sensorteil für eine Messung (57, 58, 87x, 87y, 88x, 88y), das ausgebildet ist, um einen Zustand des sich bewegenden Garns (Y) zu messen, an zumindest einem der Halter (55, 56) befestigt ist,
das zumindest eine Substrat (52, 52x, 52y) dem Halter (55, 56) zugeordnet ist, auf dem das Sensorteil für eine Messung (57, 58, 87x, 87y, 88x, 88y) befestigt ist, und das zumindest eine Substrat (52x, 52y) ausgebildet ist, um ein Signal zu verarbeiten, das von dem Sensorteil für eine Messung (57, 58, 87x, 87y, 88x, 88y), das an dem Halter (55, 56) befestigt ist, empfangen wird.
das Sensorteil für eine Messung (57, 58, 87x, 87y, 88x, 88y) über ein flexibles Verbindungsteil (73, 93) mit dem zumindest einen Substrat (52, 52x, 52y) elektrisch verbunden ist und
zwei benachbarte Halter (55, 56; 55, 156) von den Haltern (8, 55, 56; 8, 55, 156) lösbar miteinander gekoppelt sind mit einer Eingriffnahme von konkaven und konvexen Teilen.

2. Die Garnüberwachungsvorrichtung (50) gemäß Anspruch 1,
bei der das zumindest eine Substrat (52, 52x, 52y) eines einer Mehrzahl von Substraten (52, 52x, 52y) ist,
wobei das Gehäuse (54) die Halter (8, 55, 56) und die Substrate (52, 52x, 52y) aufnimmt,
wobei ein Sensorteil für eine Messung (57, 58, 87x, 87y, 88x, 88y), das ausgebildet ist, um einen Zustand des sich bewegenden Garns (Y) zu messen, an jedem der Halter (55, 56) befestigt ist,
wobei zumindest eines der Mehrzahl von Substraten (52, 52x, 52y) jedem der Halter (55, 56) zugeordnet ist und jedes der Substrate (52x, 52y) ausgebildet ist, um ein Signal zu verarbeiten, das von dem Sensorteil für eine Messung (57, 58, 87x, 87y, 88x, 88y), das an dem zugeordneten Halter (55, 56) befestigt ist, empfangen wird und
das Sensorteil für eine Messung (57, 58, 87x, 87y, 88x, 88y) in jedem der Halter (55, 56) über das flexible Verbindungsteil (73, 93) mit dem zugeordneten zumindest einen Substrat (52, 52x, 52y) elektrisch verbunden ist.

3. Die Garnüberwachungsvorrichtung (150) gemäß Anspruch 1, bei der:
eine Teilmenge der Halter (8, 55, 156) ein Sensorbefestigungshalter (55) ist, an dem ein Sensorteil für eine Messung (57, 58, 87x, 87y, 88x, 88y), das ausgebildet ist, um einen Zustand des sich bewegenden Garns (Y) zu messen, befestigt ist,
eine verbleibende Teilmenge der Halter (8, 55, 156) ein Nicht-Sensorbefestigungshalter (156) ist, an dem kein Sensorteil für eine Messung befestigt ist und
zumindest ein Substrat (52, 52x, 52y) jedem der Sensorbefestigungshalter (55) zugeordnet ist und jedes Substrat (52x, 52y) ausgebildet ist, um ein Signal zu verarbeiten, das von dem Sensorteil für eine Messung (57, 58, 87x, 87y, 88x, 88y), das an dem zugeordneten Sensorbefestigungshalter (55) befestigt ist, empfangen wird.

4. Die Garnüberwachungsvorrichtung (50; 150) gemäß einem der Ansprüche 1 bis 3, bei der
das Gehäuse (54) zumindest einen ersten Gehäuseabschnitt (105) und einen zweiten Gehäuseabschnitt (106) umfasst, der mit dem ersten Gehäuseabschnitt (105) koppelbar ist und
das Gehäuse (54) die Halter (8, 55, 56; 8, 55, 156) in einem gekoppelten Zustand aufnimmt, in dem der zweite Gehäuseabschnitt (106) mit dem ersten Gehäuseabschnitt (105) gekoppelt ist.

5. Die Garnüberwachungsvorrichtung (50; 150) gemäß Anspruch 4, bei der
ein Endhalter (55, 56; 55, 156) von den Haltern (8, 55, 56; 8, 55, 156), der an einem Ende in der vorbestimmten Richtung angeordnet ist, ein erstes Positionierungsbauglied (66, 83) umfasst, das ausgebildet ist, um den Endhalter (55, 56; 55; 156) in Bezug auf das Gehäuse (54) zu positionieren,
das Gehäuse (54) ein zweites Positionierungsbauglied (110, 115) umfasst, das ausgebildet ist, um den Endhalter (55, 56; 55; 156) in Bezug auf das Gehäuse (54) direkt oder indirekt zu positionieren und
in dem gekoppelten Zustand, die Halter (8, 55, 56; 8, 55, 156) durch den ersten Gehäuseabschnitt (105) und den zweiten Gehäuseabschnitt (106) in einer Richtung parallel zu der vorbestimmten Richtung gehalten werden und die Halter (8, 55, 56; 8, 55, 156) durch das erste Positionierungsbauglied (66, 83) und das zweite Positionierungsbauglied (110, 115) in dem Gehäuse (54) positioniert werden.

6. Die Garnüberwachungsvorrichtung (50; 150) gemäß Anspruch 5, die ferner eine Mehrzahl von Garnwegführungen (53) aufweist, die das sich bewegende Garn (Y) führen, wobei
zumindest eine Endgarnwegführung (53), die an einem Ende in der vorbestimmten Richtung zwischen den Garnwegführungen (53) angeordnet ist, zwischen den Endhaltern (55, 56; 55; 156) und dem Gehäuse (54) angeordnet ist,
die Garnwegführungen (53) ein drittes Positionierungsbauglied (104) umfassen, das ausgebildet ist, um die Garnwegführungen (53) in Bezug auf das Gehäuse (54) und die Halter (8, 55, 56; 8, 55, 156) zu positionieren,
die Halter (8, 55, 56; 8, 55, 156), das Gehäuse (54) und die Garnwegführungen (53) durch das erste Positionierungsbauglied (66, 83), das zweite Positionierungsbauglied (110, 115) und das dritte Positionierungsbauglied (104) positioniert werden.

7. Die Garnüberwachungsvorrichtung (50; 150) gemäß Anspruch 6, bei der:
entweder das erste Positionierungsbauglied (66, 83) oder das zweite Positionierungsbauglied (110, 115) ein konvexes Teil ist,
das andere des ersten Positionierungsbauglieds (66, 83) und des zweiten Positionierungsbauglieds (110, 115) eine Kerbe ist, in der das konvexe Teil Eingriff nimmt und
das dritte Positionierungsbauglied (104) ein Durchgangsloch zum Einfügen des konvexen Teils oder ein Ausschnittabschnitt ist, in dem das konvexe Teil Eingriff nimmt.

8. Die Garnüberwachungsvorrichtung (50; 150) gemäß einem der Ansprüche 1 bis 7, die ferner ein starres flexibles Substrat (70, 90) aufweist, das sich von den Substraten (52, 52x, 52y) unterscheidet, das eine Mehrzahl von starren Teilen (71, 71x, 71y, 71z, 91, 91x, 91y, 91z, 91w) und eine Mehrzahl von flexiblen Teilen (72, 92) umfasst, wobei
das Sensorteil für eine Messung (57, 58, 87x, 87y, 88x, 88y) an jedem der starren Teile (71, 71x, 71y, 71z, 91, 91x, 91y, 91z, 91w) befestigt ist,
die flexiblen Teile (72, 92) zumindest zwei der starren Teile (71, 71x, 71y) elektrisch miteinander verbinden und
die starren Teile (71, 71x, 71y, 71z, 91, 91x, 91y, 91z, 91w) und die flexiblen Teile (72, 92) so angeordnet sind, dass dieselben von der vorbestimmten Richtung aus gesehen einen Bewegungsbereich des Garns (Y) umgeben.

9. Die Garnüberwachungsvorrichtung (50; 150) gemäß einem der Ansprüche 1 bis 8, bei der die Halter (8, 55, 56; 8, 55, 156) folgende Merkmale umfassen:
einen ersten Halter (55), der ein erstes Erfassungsmodul (M1) bildet, das zumindest eine Funktion aufweist, eine Dicke des Garns (Y) zu erfassen und
einen zweiten Halter (56), der ein zweites Erfassungsmodul (M2) bildet, das zumindest eine Funktion aufweist, eine Fremdsubstanz zu erfassen, die in dem Garn (Y) enthalten ist.

10. Die Garnüberwachungsvorrichtung (50; 150) gemäß einem der Ansprüche 1 bis 9, bei der das Substrat (52, 52x, 52y) ein flaches Substrat ist.

11. Garnwickelmaschine (1), die ausgebildet ist, ein Garn (Y) zu wickeln, um einen Wickelkörper (P) zu bilden, die die Garnüberwachungsvorrichtung (50; 150) gemäß einem der Ansprüche 1 bis 9 aufweist, wobei die vorbestimmte Richtung eine Bewegungsrichtung des Garns (Y) ist.

## Revendications

1. Dispositif de surveillance de fil (50), comprenant:
une pluralité de supports (8, 55, 56) qui sont empilés l'un au-dessus de l'autre dans une direction prédéterminée et à travers lesquels passe un fil (Y) en déplacement;
au moins un substrat (52, 52x, 52y); et
un boîtier (54) qui abrite les supports (8, 55, 56) et l'au moins un substrat (52, 52x, 52y),
dans lequel
un élément capteur pour la mesure (57, 58, 87x, 87y, 88x, 88y) qui est configuré pour mesurer un état du fil en déplacement (Y) est monté sur au moins l'un des supports (55, 56),
l'au moins un substrat (52, 52x, 52y) est associé au support (55, 56) sur lequel est monté l'élément capteur pour une mesure (57, 58, 87x, 87y, 88x, 88y), et au moins un le substrat (52x, 52y) est configuré pour traiter un signal reçu de l'élément capteur pour une mesure (57, 58, 87x, 87y, 88x, 88y) montée sur le support (55, 56),
l'élément capteur pour la mesure (57, 58, 87x, 87y, 88x, 88y) est connecté électriquement à l'au moins un substrat (52, 52x, 52y) par l'intermédiaire d'un élément de connexion flexible (73, 93), et
deux supports adjacents (55, 56; 55, 156) parmi les supports (8, 55, 56; 8, 55, 156) sont couplés de manière amovible l'un à l'autre avec venue en prise des parties concave et convexe.

2. Dispositif de surveillance de fil (50) selon la revendication 1,
dans lequel l'au moins un substrat (52, 52x, 52y) est l'un d'une pluralité de substrats (52, 52x, 52y),
dans lequel le boîtier (54) abrite les supports (8, 55, 56) et les substrats (52, 52x, 52y),
dans lequel un élément capteur pour la mesure (57, 58, 87x, 87y, 88x, 88y) qui est configuré pour mesurer un état du fil en déplacement (Y) est monté sur chacun des supports (55, 56),
dans lequel au moins un de la pluralité de substrats (52, 52x, 52y) est associé à chacun des supports (55, 56) et chacun des substrats (52x, 52y) est configuré pour traiter un signal reçu de l'élément capteur pour la mesure (57, 58, 87x, 87y, 88x, 88y) monté sur le support associé (55, 56), et
l'élément capteur pour la mesure (57, 58, 87x, 87y, 88x, 88y) dans chacun des supports (55, 56) est connecté électriquement à l'au moins un substrat associé (52, 52x, 52y) via l'élément de connexion flexible (73, 93).

3. Dispositif de surveillance de fil (150) selon la revendication 1, dans lequel:
un sous-ensemble des supports (8, 55, 156) est un support de montage de capteur (55) sur lequel est monté un élément capteur pour la mesure (57, 58, 87x, 87y, 88x, 88y) qui est configuré pour mesurer un état de déplacement le fil (Y),
un sous-ensemble restant des supports (8, 55, 156) est un support sans montage de capteur (156) sur lequel n'est pas monté d'élément capteur pour la mesure, et
au moins un substrat (52, 52x, 52y) est associé à chacun des supports de montage de capteur (55) et chaque substrat (52x, 52y) est configuré pour traiter un signal reçu de l'élément capteur pour la mesure (57, 58, 87x, 87y, 88x, 88y) monté sur le support de montage de capteur associé (55).

4. Dispositif de surveillance de fil (50; 150) selon l'une quelconque des revendications 1 à 3, dans lequel
le boîtier (54) comporte au moins un premier segment de boîtier (105) et un deuxième segment de boîtier (106) pouvant être couplé au premier segment de boîtier (105), et
le boîtier (54) abrite les supports (8, 55, 56; 8, 55, 156) dans un état couplé dans lequel le deuxième segment de boîtier (106) est couplé au premier segment de boîtier (105).

5. Dispositif de surveillance de fil (50; 150) selon la revendication 4, dans lequel
un support d'extrémité (55, 56; 55, 156) parmi les supports (8, 55, 56; 8, 55, 156) disposé à une extrémité dans la direction prédéterminée comporte un premier élément de positionnement (66, 83) qui est configuré pour positionner le support d'extrémité (55, 56; 55, 156) par rapport au boîtier (54),
le boîtier (54) comporte un deuxième élément de positionnement (110, 115) qui est configuré pour positionner directement ou indirectement le support d'extrémité (55, 56; 55, 156) par rapport au boîtier (54), et
à l'état couplé, les supports (8, 55, 56; 8, 55, 156) sont maintenus par le premier segment de boîtier (105) et le deuxième segment de boîtier (106) dans une direction parallèle à la direction prédéterminée et les supports (8, 55, 56, 8, 55, 156) sont positionnés dans le boîtier (54) par le premier élément de positionnement (66, 83) et le deuxième élément de positionnement (110, 115).

6. Dispositif de surveillance de fil (50; 150) selon la revendication 5, comprenant par ailleurs une pluralité de guide-trajet de fil (53) qui guident le fil en déplacement (Y), dans lequel
au moins un guide-trajet de fil d'extrémité (53) disposé à une extrémité dans la direction prédéterminée parmi les guide-trajet de fil (53) est disposé entre les supports d'extrémité (55, 56; 55, 156) et le boîtier (54),
les guide-trajet de fil (53) comportent un troisième élément de positionnement (104) qui est configuré pour positionner les guide-trajet de fil (53) par rapport au boîtier (54) et aux supports (55, 56; 55, 156),
les supports (8, 55, 56; 8, 55, 156), le boîtier (54) et les guide-trajet de fil (53) sont positionnés par le premier élément de positionnement (66, 83), le deuxième élément de positionnement (110, 115) et le troisième élément de positionnement (104).

7. Dispositif de surveillance de fil (50; 150) selon la revendication 6, dans lequel
l'un parmi le premier élément de positionnement (66, 83) et le deuxième élément de positionnement (110, 115) est un élément convexe,
l'autre parmi le premier élément de positionnement (66, 83) et le deuxième élément de positionnement (110, 115) est une encoche dans laquelle s'engage l'élément convexe, et
le troisième élément de positionnement (104) est un trou traversant destiné à insérer l'élément convexe, ou une partie découpée dans laquelle s'engage l'élément convexe.

8. Dispositif de surveillance de fil (50; 150) selon l'une quelconque des revendications 1 à 7, comprenant par ailleurs un substrat flexible rigide (70, 90), qui est différent des substrats (52, 52x, 52y), comportant une pluralité de parties rigides (71, 71x, 71y, 71z, 91, 91x, 91y, 91z, 91w) et une pluralité de parties flexibles (72, 92), dans lequel
l'élément capteur pour la mesure (57, 58, 87x, 87y, 88x, 88y) est monté sur chacune des parties rigides (71, 71x, 71y, 71z, 91, 91x, 91y, 91z, 91w),
les parties flexibles (72, 92) connectent électriquement l'une à l'autre au moins deux des parties rigides (71, 71x, 71y, 71z, 91, 91x, 91y, 91z, 91w), et
les parties rigides (71, 71x, 71y, 71z, 91, 91x, 91y, 91z, et les parties flexibles (72, 92) sont disposées de manière à entourer une zone de déplacement du fil (Y), lorsque vue depuis la direction prédéterminée.

9. Dispositif de surveillance de fil (50; 150) selon l'une quelconque des revendications 1 à 8, dans lequel
les supports (8, 55, 56; 8, 55, 156) comportent
un premier support (55) qui constitue un premier module de détection (M1) ayant au moins une fonction de détection d'une épaisseur du fil (Y), et
un deuxième support (56) qui constitue un deuxième module de détection (M2) ayant au moins une fonction de détection d'une substance étrangère contenue dans le fil (Y).

10. Dispositif de surveillance de fil (50; 150) selon l'une quelconque des revendications 1 à 9, dans lequel le substrat (52, 52x, 52y) est un substrat plat.

11. Machine de bobinage de fil (1) configurée pour bobiner un fil (Y) pour former un paquet (P), comprenant le dispositif de surveillance de fil (50; 150) selon l'une quelconque des revendications 1 à 9, dans laquelle la direction prédéterminée est une direction de déplacement du fil (Y).
